(19) Europäisches Patentamt
European Patent Office
Office européen des brevets

(11) EP 3 134 518 B1

(12) # FASCICULE DE BREVET EUROPEEN

(45) Date de publication et mention de la délivrance du brevet:
**10.06.2020 Bulletin 2020/24**

(21) Numéro de dépôt: **15726231.2**

(22) Date de dépôt: **22.04.2015**

(51) Int Cl.:
***C12N 9/02*** *(2006.01)* ***C12Q 1/26*** *(2006.01)*

(86) Numéro de dépôt international:
**PCT/FR2015/051101**

(87) Numéro de publication internationale:
**WO 2015/162383 (29.10.2015 Gazette 2015/43)**

(54) **PROTEINES NADPH OXYDASES**

NADPH-OXIDASEPROTEINE

NADPH OXIDASE PROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.04.2014 FR 1453616**

(43) Date de publication de la demande:
**01.03.2017 Bulletin 2017/09**

(73) Titulaires:
- **Université Grenoble Alpes
38400 Saint Martin d'Hères (FR)**
- **Centre National de la Recherche Scientifique
75794 Paris Cedex 16 (FR)**

(72) Inventeurs:
- **DUPUY, Jérôme
38210 Saint Quentin sur Isère (FR)**
- **HAJJAR, Christine
Agence Machghara, Bekaa (LB)**
- **CHERRIER, Mickaël
38210 Tullins (FR)**
- **FIESCHI, Franck
38450 Vif (FR)**

(74) Mandataire: **Ipside
6, Impasse Michel Labrousse
31100 Toulouse (FR)**

(56) Documents cités:
**WO-A1-01/00668 WO-A1-2005/058942
WO-A2-02/077183 WO-A2-2004/011670
WO-A2-2011/062864**

- **DATABASE UniProt [Online] 29 mai 2013 (2013-05-29), "SubName: Full=Oxidoreductase NAD-binding domain protein {ECO:0000313|EMBL:ELU89002.1};", XP002733520, extrait de EBI accession no. UNIPROT:M5ML45 Database accession no. M5ML45**
- **DATABASE UniProt [Online] 22 janvier 2014 (2014-01-22), "SubName: Full=Uncharacterized protein {ECO:0000313|EMBL:ERZ30597.1};", XP002733521, extrait de EBI accession no. UNIPROT:U9QZD2 Database accession no. U9QZD2**
- **DATABASE UniProt [Online] 19 février 2014 (2014-02-19), "SubName: Full=Ferric reductase-like transmembrane component {ECO:0000313|EMBL:ETG99185.1};", XP002733522, extrait de EBI accession no. UNIPROT:V8TZ76 Database accession no. V8TZ76**

Remarques:
Le document complet y compris le(s) table(s) de référence et le(s) listage(s) de séquence sont téléchargeables sur le site internet de l'OEB.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 134 518 B1

## Description

La présente invention concerne l'utilisation pour le criblage de molécules présentant un intérêt thérapeutique de protéines NADPH oxydases.

Les protéines NADPH oxydases, ou Nox, sont des protéines modulaires tissu-spécifiques très répandues chez les eucaryotes. Elles partagent un cœur catalytique consistant en un domaine FNR (*flavin-containing ferredoxin NADPH oxidoreductase-like*) et un domaine FRD (*ferric reductase domain*), lié à la membrane, fixant 2 hèmes (Nox 1 à 4). Elles peuvent également posséder, comme Nox5, un domaine calmoduline et, en outre, comme dans le cas de Duoxl et Duox2, un segment transmembranaire supplémentaire et un domaine de type peroxydase en N-terminal (Lambeth JD, NOX enzymes and the biology of reactive oxygen, Nature reviews, 2004, Immunology, 4:181-189). Bien que les deux domaines FNR et cytochrome b soient retrouvés chez les procaryotes, les alignements purs et simples de séquences n'ont jamais permis de détecter de protéines Nox ou Fre dans les génomes bactériens.

En termes de fonction, en plus de Nox2, qui est trouvée à la fois dans les phagocytes, où elle intervient dans la défense immunitaire, et dans les tissus cardiaques, où elle joue un rôle dans la contraction musculaire, les enzymes Nox interviennent dans de multiples processus tels que la prolifération cellulaire, l'apoptose ou encore la communication intra et intercellulaire. Par ailleurs, les enzymes Duox interviennent dans la synthèse des hormones thyroïdiennes (Prosser BL et al., X-ROS signaling : rapid mechano-chemo transduction in heart, 2011, Science, 333:1440-1445 ; De Deken X et al., Cloning of two human thyroid cDNAs encoding new members of the NADPH oxidase family, The Journal of biological chemistry, 275:23227-23233). Chez la levure, les réductases ferriques (Fre) sont impliquées, quant à elles, dans la réduction des ions ferriques en ions ferreux au cours de l'importation du fer.

De manière régulée, les protéines Nox aboutissent à la production d'espèces réactives de l'oxygène ou ROS (Reactive Oxygen species), tels que les ions superoxyde $O_2^{\bullet -}$, le peroxyde d'hydrogène ($H_2O_2$) ou encore le radical hydroxyle $OH^{\bullet}$. L'accumulation récente de données indique que les ROS ont un rôle beaucoup plus subtil que celui initialement attribué dans le système immunitaire. Leur importance dans des mécanismes moléculaires et cellulaires comme la signalisation, la réactivité chimique ou encore la toxicité induite par le potentiel oxydo-réducteur des espèces produites place donc désormais les protéines Nox au rang de cibles thérapeutiques à forte valeur ajoutée.

Pour identifier de nouveaux médicaments susceptibles de cibler spécifiquement cette famille de protéines, l'industrie pharmaceutique effectue des criblages de petites molécules pour déterminer leur impact vis-à-vis des protéines Nox. Ces petites molécules sont ensuite améliorées par synthèse organique afin d'optimiser leurs effets.

Or, compte tenu de l'origine eucaryote des protéines Nox, de leur qualité de protéines membranaires, de leurs modes de régulation, et de leurs modifications post-traductionnelles spécifiques (Nox1-4, Nox5 et Duoxl-2), les recherches pharmaceutiques actuelles doivent faire face aux difficultés inhérentes à la nature même de ces protéines Nox. En d'autres termes, il est difficile de produire en quantités suffisantes un système membranaire glycosylé, à composants multiples et hautement régulé.

Pour réaliser des essais de criblage, il est pourtant nécessaire d'une part, d'obtenir des protéines Nox fonctionnelles pour évaluer l'impact effectif des molécules testées, et d'autre part, de produire des quantités suffisantes de protéines Nox pour pouvoir tester un grand nombre de molécules. Or, les protéines Nox produites en quantités importantes sont principalement inactives alors que les protéines Nox fonctionnelles sont, quant à elles, obtenues en quantités insignifiantes. Ceci a pour effet de rendre leur coût de production trop élevé pour envisager des essais de criblage à grande échelle.

A l'heure actuelle, des protéines Nox humaines ont déjà été produites dans des cellules du système immunitaire (Taylor RM et al., Analysis of human phagocyte flavocytochrome b(558) by mass spectrometry, J Biol Chem 2006, Dec 1, 281(48) : 37045-56 ; Lord CI et al., Single-step immunoaffinity purification and functional reconstitution of human phagocyte flavocytochrome b, J Immunol Methods, 2008, Jan 1, 329(1-2):201-7) et différentes tentatives d'expression hétérologue ont également été envisagées, mais sans succès (de Mendez I, Leto TL, Functional reconstitution of the phagocyte NADPH oxidase by transfection of its multiple components in a heterologous system, Blood, 1995, Feb 15, 85(4):1104-10 ; Price MO et al. Creation of a genetic system for analysis of the phagocyte respiratory burst: high-level reconstitution of the NADPH oxidase in a nonhematopoietic system. Blood, 2002, Apr 15, 99(8):2653-61; Zhen L et al., Gene targeting of X chromosome-linked chronic granulomatous disease locus in a human myeloid leukemia cell line and rescue by expression of recombinant gp91phox. Proc Natl Acad Sci USA, 1993 Nov 1, 90(21):9832-6).

Des protéines Nox de mammifères ont notamment été exprimées de manière hétérologue chez différents organismes eucaryotes, comme dans des cellules d'insectes (Rotrosen D et al., Production of recombinant cytochrome b558 allows reconstitution of the phagocyte NADPH oxidase solely from recombinant proteins, J Biol Chem, 1993, Jul 5, 268(19):14256-60) ou chez la levure (Ostuni MA et al., Expression of functional mammal flavocytochrome b(558) in yeast : comparison with improved insect cell system. Biochim Biophys Acta, 2010 Jun, 1798(6):1179-88).

Toutefois, aucune de ces approches n'a permis de produire des protéines Nox fonctionnelles en quantités suffisantes pour envisager du criblage à haut débit.

Par ailleurs, des systèmes d'expression hétérologue ont également été testés pour tenter de produire des

protéines Nox eucaryotes chez des organismes procaryotes, en particulier chez la bactérie *Escherichia coli,* mais seules des formes tronquées des protéines Nox eucaryotes ont été produites (Han CH et al., Characterization of the flavoprotein domain of gp91phox which has NADPH diaphorase activity, J Biochem, 2001 Apr, 129(4):513-20 ; Nisimoto Y et al. Activation of the flavoprotein domain of gp91phox upon interaction with N-terminal p67phox (1-210) and the Rac complex, Biochemistry, 2004, Jul 27, 43(29):9567-75).

Dans ce contexte, la présente invention a pour but de proposer de l'utilisation de protéines Nox, identifiées et caractérisées pour la première fois chez les procaryotes. Le recours à ces protéines Nox présente ainsi l'avantage de permettre une production de protéines fonctionnelles en quantité importante et à moindre coût en comparaison des protéines Nox eucaryotes.

L'invention concerne l'utilisation telle que définie dans la revendication 1, *in vitro, ex vivo* ou *in vivo,* comme outil de recherche pour le criblage de molécules présentant un intérêt thérapeutique, d'une protéine Nox (ou NADPH oxydase) ayant :

- un domaine comprenant de 3 à 7 hélices transmembranaires, et possédant une séquence en acides aminés comprenant :
- au moins 2 motifs bis-histidyl, chacun des motifs bis-histidyl étant constitué de 2 résidus histidine séparés par 12, 13 ou 14 résidus d'acides aminés, lesdits 2 motifs bis-histidyl étant situés dans la partie membranaire et séparés par une hélice transmembranaire, et
- au moins un premier motif constitué de la séquence :
  [G/S/A]-[Q/D]-F-[A/V/T/L/F]-[F/Y/W/L/R]-[L/V/I/F/W] (SEQ ID NO : 1), et
- au moins un deuxième motif constitué de la séquence :
  [P/A/S/E/F]-H-[P/S/A]-F-[T/S]-[L/I/M/V] (SEQ ID NO : 2), et
- au moins un troisième motif constitué de la séquence :
  [K/R]-X-X-G-[D/G]-X-[T/S] (SEQ ID NO : 3), X représentant n'importe quel acide aminé naturel, et
- au moins un quatrième motif constitué de la séquence :
  G-[I/S/V/L]-G-[V/I/A/F]-[T/A/S]-[P/Y/T/A] (SEQ ID NO : 4),

ladite protéine Nox n'étant pas utilisée *in vivo* chez l'homme.

Dans l'invention, le terme "Nox", ou "protéine NADPH oxydase" fait référence à une enzyme catalysant la réaction de transfert des électrons d'un donneur d'électrons, homologue du NADPH, à un accepteur final qui est l'oxygène moléculaire. Il en résulte la production d'espèces réactives de l'oxygène.

Cette activité NADPH oxydase des protéines Nox intervient naturellement de manière *in vivo* chez les organismes qui les produisent, mais elle peut également être mise en évidence et mesurée *in vitro* à l'aide de tests spécifiques, par l'intermédiaire de réactions de réduction montrant la production d'ion superoxyde (par colorimétrie en suivant la réduction du cytochrome c, ou la réduction du NBT (Nitro Blue Tetrazolium), ou aussi par fluorescence en utilisant l'amplex Red, et par chimiluminescence en utilisant le luminol).

Le terme "partie membranaire" fait référence aux acides aminés qui se retrouvent disposés dans la bicouche lipidique de la cellule, lorsque la protéine Nox est exprimée de manière fonctionnelle. Cette partie membranaire contient les 2 hèmes de la protéine Nox et est essentiellement composée d'acides aminés repliés sous forme d'hélices a.

La présente invention repose sur la constatation surprenante faîte par les Inventeurs qu'il existe chez les procaryotes des protéines de la famille Nox.

Pour la première fois, les Inventeurs ont identifié des motifs présents sur la séquence d'acides aminés de ces protéines Nox, lesquels motifs permettent d'identifier et d'annoter correctement lesdites protéines Nox.

L'approche des Inventeurs repose sur l'identification (i) d'un motif consensus correspondant au domaine de fixation du FAD, (ii) d'un motif consensus correspondant au domaine de fixation du NADPH et (iii) d'un domaine comprenant de 3 à 7 hélices transmembranaires (identifiées à partir de la séquence d'acides aminés) contenant 2 motifs bis-histidyl de 12 à 14 acides aminés séparés d'au moins 20 acide aminés (soit la longueur d'une hélice transmembranaire), qui sont retrouvés en association dans des protéines Nox.

De plus, les Inventeurs ont également identifié de manière surprenante des motifs supplémentaires présents chez la plupart des Nox procaryotes, permettant de les distinguer des Nox eucaryotes.

Les protéines Nox utilisées selon l'invention peuvent ainsi être identifiées à partir de leur séquence d'acides aminés à l'aide des 4 motifs suivants :

1$^{er}$ motif : [G/S/A]-[Q/D]-F-[A/V/T/L/F]-[F/Y/W/L/R]-[L/V/I/F/W] (SEQ ID NO : 1),
2$^{ème}$ motif : [P/A/S/E/F]-H-[P/S/A]-F-[T/S]-[L/I/M/V] (SEQ ID NO : 2), ce motif correspond au site de fixation du FAD sur la protéine Nox,
3$^{ème}$ motif : [K/R]-X-X-G-[D/G]-X-[T/S] (SEQ ID NO : 3),
4$^{ème}$ motif : G-[I/S/V/L]-G-[V/I/A/F]-[T/A/S]-[P/Y/T/A] (SEQ ID NO : 4), ce motif correspond au site de fixation du

NADPH sur la protéine Nox.

Les acides aminés indiqués entre crochets correspondent à des acides aminés alternatifs pour la position considérée sur la séquence consensus.

Les protéines Nox utilisées selon l'invention correspondent à des protéines Nox identifiées chez des organismes procaryotes. Il n'est toutefois pas exclu que des protéines Nox telles que définies plus haut puissent être identifiées chez des organismes eucaryotes.

Dans un mode de réalisation, l'invention a pour objet l'utilisation d'une protéine Nox telle que définie plus haut, dans laquelle ledit premier motif est choisi parmi le groupe constitué des séquences SEQ ID NO : 8, SEQ ID NO : 9 et SEQ ID NO : 10.

Les séquences SEQ ID NO : 8 à 10 correspondent respectivement aux motifs présents sur les protéines Nox de *Streptococcus pneumoniae, Pseudomonas aeruginosa et Escherichia coli* (SEQ ID NO : 8), *Salmonella enterica* et *Bordetella pertussis* (SEQ ID NO : 9) et *Vibrio cholerae* (SEQ ID NO : 10).

Dans un mode de réalisation, l'invention concerne l'utilisation d'une protéine Nox telle que définie plus haut, dans laquelle ledit deuxième motif est choisi parmi le groupe constitué des séquences SEQ ID NO : 11, SEQ ID NO : 12, SEQ ID NO : 13 et SEQ ID NO : 14.

Les séquences SEQ ID NO : 11 à 14 correspondent respectivement aux motifs présents sur les protéines Nox de *Streptococcus pneumoniae* (SEQ ID NO: 11), *Pseudomonas aeruginosa, Salmonella enterica* et *Bordetella pertussis* (SEQ ID NO : 12), *Vibrio cholerae* (SEQ ID NO : 13) et *Escherichia coli* (SEQ ID NO : 14).

Dans un mode de réalisation, l'invention concerne l'utilisation d'une protéine NOX telle que définie plus haut, dans laquelle ledit troisième motif est choisi parmi le groupe constitué des séquences SEQ ID NO : 15, SEQ ID NO : 16, SEQ ID NO : 17, SEQ ID NO : 18, SEQ ID NO : 19 et SEQ ID NO : 20.

Les séquences SEQ ID NO : 15 à 20 correspondent respectivement aux motifs présents sur les protéines Nox de *Streptococcus pneumoniae* (SEQ ID NO: 15), *Pseudomonas aeruginosa* (SEQ ID NO : 16), *Escherichia coli* (SEQ ID NO : 17), *Vibrio cholerae* (SEQ ID NO : 18), *Salmonella enterica* (SEQ ID NO : 19) et *Bordetella pertussis* (SEQ ID NO : 20).

Dans un mode de réalisation, l'invention concerne l'utilisation d'une protéine NOX telle que définie plus haut, dans laquelle ledit quatrième motif est choisi parmi le groupe constitué des séquences SEQ ID NO : 21, SEQ ID NO : 22 et SEQ ID NO : 23.

Les séquences SEQ ID NO : 21 à 23 correspondent respectivement aux motifs présents sur les protéines Nox de *Streptococcus pneumoniae, Salmonella enterica, Escherichia coli* et *Bordetella pertussis* (SEQ ID NO : 21), *Pseudomonas aeruginosa* (SEQ ID NO : 22) et *Vibrio cholerae* (SEQ ID NO : 23).

Dans un mode de réalisation particulier, l'invention a pour objet l'utilisation d'une protéine Nox telle que définie précédemment, dans laquelle la séquence en acides aminés de la protéine Nox comprend, ou consiste en, une séquence ayant au moins 90 % d'identité, de préférence 91%, 92%, 93%, 94%, 95%, 96%, 97%, 98%, 99% ou 100% d'identité avec la SEQ ID NO : 24.

La séquence SEQ ID NO : 24 correspond à une protéine Nox identifiée chez *Streptococcus pneumoniae*.

Le *"pourcentage d'identité"* entre deux séquences d'acides aminés, au sens de la présente invention, est déterminé en comparant les deux séquences alignées de manière optimale, à travers une fenêtre de comparaison. La partie de la séquence d'acides aminés dans la fenêtre de comparaison peut ainsi comprendre des additions ou des délétions (par exemple des "gaps") par rapport à la séquence de référence (qui ne comprendrait pas ces additions ou ces délétions) de manière à obtenir un alignement optimal entre les deux séquences.

Le pourcentage d'identité est calculé en déterminant le nombre de positions pour lesquelles un résidu d'acide aminé est identique chez les deux séquences comparées, puis en divisant le nombre de positions pour lesquelles il y a identité entre les deux résidus d'acides aminés, par le nombre total de positions dans la fenêtre de comparaison, puis en multipliant le résultat par cent afin d'obtenir le pourcentage d'identité en acides aminés des deux séquences entre elles.

Dans un mode de réalisation particulier, l'invention a pour objet l'utilisation d'une protéine NOX telle que définie précédemment, dans laquelle la séquence en acides aminés de la protéine Nox comprend, ou consiste en, une séquence ayant au moins 90 % d'identité, de préférence 91%, 92%, 93%, 94%, 95%, 96%, 97%, 98%, 99% ou 100% d'identité avec la SEQ ID NO : 25.

La séquence SEQ ID NO : 25 correspond à une protéine Nox identifiée chez *Pseudomonas aeruginosa*.

Dans un mode de réalisation particulier, l'invention a pour objet l'utilisation d'une protéine NOX telle que définie précédemment, dans laquelle la séquence en acides aminés de la protéine Nox comprend, ou consiste en, une séquence ayant au moins 90 % d'identité, de préférence 91%, 92%, 93%, 94%, 95%, 96%, 97%, 98%, 99% ou 100% d'identité avec la SEQ ID NO : 26.

La séquence SEQ ID NO : 26 correspond à une protéine Nox identifiée chez *Escherichia coli*.

Dans un mode de réalisation particulier, l'invention a pour objet l'utilisation d'une protéine Nox telle que définie précédemment, dans laquelle la séquence en acides aminés de la protéine Nox comprend, ou consiste en, une séquence

ayant au moins 90 % d'identité, de préférence 91%, 92%, 93%, 94%, 95%, 96%, 97%, 98%, 99% ou 100% d'identité avec la SEQ ID NO : 27.

La séquence SEQ ID NO: 27 correspond à une protéine Nox identifiée chez *Vibrio cholerae.*

Dans un mode de réalisation particulier, l'invention a pour objet l'utilisation d'une protéine Nox telle que définie précédemment, dans laquelle la séquence en acides aminés de la protéine Nox comprend, ou consiste en, une séquence ayant au moins 90 % d'identité, de préférence 91%, 92%, 93%, 94%, 95%, 96%, 97%, 98%, 99% ou 100% d'identité avec la SEQ ID NO : 28.

La séquence SEQ ID NO : 28 correspond à une protéine Nox identifiée chez *Salmonella enterica.*

Dans un mode de réalisation particulier, l'invention a pour objet l'utilisation d'une protéine Nox telle que définie précédemment, dans laquelle la séquence en acides aminés de la protéine Nox comprend, ou consiste en, une séquence ayant au moins 90 % d'identité, de préférence 91%, 92%, 93%, 94%, 95%, 96%, 97%, 98%, 99% ou 100% d'identité avec la SEQ ID NO : 29.

La séquence SEQ ID NO : 29 correspond à une protéine Nox identifiée chez *Bordetella pertussis.*

Dans un mode de réalisation, l'invention concerne l'utilisation d'une protéine Nox telle que définie plus haut, dans laquelle la séquence en acides aminés de la protéine Nox comprend, ou consiste en, une séquence choisie parmi le groupe constitué des séquences SEQ ID NO : 24 à 351.

Les séquences SEQ ID NO : 24 à 351 correspondent à 328 séquences de protéines Nox identifiées parmi les séquences accessibles dans les bases de données au 22 janvier 2014.

Dans un mode de réalisation, l'invention concerne l'utilisation d'une protéine NOX telle que définie précédemment, dans laquelle la séquence en acides aminés de la protéine Nox comprend, ou consiste en, une séquence choisie parmi le groupe constitué des séquences SEQ ID NO : 24, SEQ ID NO : 25, SEQ ID NO : 26, SEQ ID NO : 27, SEQ ID NO : 28 et SEQ ID NO : 29.

Dans un mode de réalisation particulier, ladite protéine Nox est une protéine d'un organisme procaryote, ledit organisme procaryote étant de préférence choisie parmi le groupe constitué de *Streptococcus pneumoniae, Pseudomonas aeruginosa, Escherichia coli, Vibrio cholerae, Salmonella enterica* et *Bordetella pertussis.*

Dans un autre mode de réalisation particulier, ladite protéine Nox est une protéine isolée.

Dans un autre mode de réalisation particulier, ladite protéine Nox est une protéine recombinante.

Dans l'invention, le terme « protéine recombinante » fait référence à une protéine produite dans une cellule dans laquelle le gène codant pour cette protéine a été introduit, comme par exemple à l'aide d'un vecteur plasmidique. Une protéine recombinante peut être produite de manière hétérologue dans une cellule d'un organisme différent de celui dans lequel elle a été identifiée à l'origine.

Dans un autre mode de réalisation particulier, ladite protéine Nox possède une signature spectrale en spectrophotométrie de 250 à 700 nm similaire, de préférence identique, à l'une des signatures spectrales de 250 à 700 nm présentées dans la figure 5.

La présence des hèmes dans la protéine Nox est en effet visualisable en effectuant une mesure par la technique de spectrophotométrie. La présence des hèmes induit une signature spectrale caractéristique de 250 à 700 nm, avec un pic à 410 nm lorsque la protéine est dans son état oxydé. Un autre spectre caractéristique des Nox est le spectre de différence (spectre réduit moins spectre oxydé) présentant un pic majeur à 425nm et un autre pic à 558nm. Ainsi, l'analyse de la signature spectrale permet de visualiser la présence des hèmes et par conséquent, de vérifier le bon repliement et la stabilité de la protéine Nox analysée.

Dans un autre mode de réalisation particulier, l'invention concerne l'utilisation d'une protéine Nox telle que définie précédemment, ladite protéine Nox possédant une activité NADPH oxydase entraînant une production de ROS en présence d'un donneur d'électrons, ladite activité NADPH oxydase étant susceptible d'être détectée dans un échantillon comprenant ladite protéine Nox par l'intermédiaire d'une réaction montrant la production d'ion superoxyde, de préférence par une réduction du cytochrome c ou par une réduction du NBT (Nitro Blue Tetrazolium), et de préférence à l'aide d'une mesure d'absorbance par spectrophotométrie, par chimiluminescence ou par un test colorimétrique, réalisés à partir de l'échantillon.

Dans un autre aspect, l'invention concerne également l'utilisation *in vitro, ex vivo* ou *in vivo,* d'un acide nucléique codant une protéine Nox définie précédemment comme outil de recherche pour le criblage de molécules présentant un intérêt thérapeutique, ledit acide nucléique n'étant pas utilisé *in vivo* chez l'homme.

Dans un mode réalisation, ledit acide nucléique comprend, ou consiste en, une séquence choisie parmi le groupe constitué des séquences SEQ ID NO : 352, SEQ ID NO : 353, SEQ ID NO : 354, SEQ ID NO : 355, SEQ ID NO : 356, SEQ ID NO : 357, SEQ ID NO : 358, SEQ ID NO : 359, SEQ ID NO : 360, SEQ ID NO : 361, SEQ ID NO : 362 et SEQ ID NO : 363.

Les séquences SEQ ID NO : 352 à 357 correspondent à des acides nucléiques codant des protéines Nox identifiées chez *S. pneumoniae* (SEQ ID NO : 352), *P. aeruginosa* (SEQ ID NO : 353), *E. coli* (SEQ ID NO : 354), *V. cholerae* (SEQ ID NO : 355), *S. enterica* (SEQ ID NO : 356) et *B. pertussis* (SEQ ID NO : 357).

Les séquences SEQ ID NO : 358 à 363 correspondent à des acides nucléiques, dont les codons ont été

optimisés pour permettre l'expression de protéines Nox de *S. pneumoniae* (SEQ ID NO : 358), *P. aeruginosa* (SEQ ID NO : 359), *E. coli* (SEQ ID NO : 360), V. *cholerae* (SEQ ID NO : 361), *S. enterica* (SEQ ID NO : 362) et *B. pertussis* (SEQ ID NO : 363), possédant une queue poly-histidine et un site de coupure à la thrombine.

Dans un autre aspect, l'invention concerne l'utilisation *in vitro, ex vivo* ou *in vivo,* d'un vecteur d'expression comprenant un acide nucléique tel que défini ci-dessus, comme outil de recherche pour le criblage de molécules présentant un intérêt thérapeutique, ledit vecteur d'expression n'étant pas utilisé *in vivo* chez l'homme.

Dans un mode de réalisation, ledit vecteur d'expression tel que défini ci-dessus comprend, ou consiste en, une séquence choisie parmi le groupe constitué des séquences SEQ ID NO : 364, SEQ ID NO : 365, SEQ ID NO : 366, SEQ ID NO : 367, SEQ ID NO : 368 et SEQ ID NO : 369.

Dans un autre aspect, l'invention concerne l'utilisation *in vitro, ex vivo* ou *in vivo,* d'une cellule contenant au moins un vecteur d'expression tel que défini précédemment, comme outil de recherche pour le criblage de molécules présentant un intérêt thérapeutique, ladite cellule n'étant pas utilisée *in vivo* chez l'homme.

Dans un autre mode de réalisation, la dite cellule est une bactérie, de préférence choisie parmi le groupe constitué d'*Escherichia coli, Streptococcus pneumoniae, Pseudomonas aeruginosa, Vibrio cholerae, Salmonella enterica* et *Bordetella pertussis.*

Un procédé de préparation d'une protéine Nox telle que définie plus haut, à partir d'un extrait cellulaire de cellules produisant ladite protéine, contenu dans une solution appropriée, comprend une étape de solubilisation des membranes cellulaires dudit extrait cellulaire pour mettre ladite protéine en suspension dans ladite solution appropriée.

Compte tenu de leur structure complexe et de leur nature membranaire, les protéines Nox sont difficiles à produire et à purifier car il est difficile de solubiliser la membrane pour libérer la protéine Nox sans déstabiliser sa structure. L'utilisation d'un détergent non adapté entraîne d'ailleurs une dégradation de la protéine Nox.

Toutefois, les Inventeurs ont constaté de manière surprenante que l'utilisation de détergents de type maltoside permet de solubiliser la protéine Nox.

Dans le procédé tel que défini ci-dessus, l'étape de solubilisation de ladite membrane cellulaire peut être réalisée à l'aide d'au moins un détergent de la famille des maltosides, de préférence choisi parmi le groupe des dodecyl-maltosides.

Le détergent va ainsi tapisser la partie membranaire en remplaçant les lipides membranaire. Le détergent va alors maintenir la structure tridimensionnelle de la protéine Nox qui va maintenir les hèmes, lors de la purification, dans la partie membranaire.

Dans un procédé tel que défini ci-dessus, ledit au moins un détergent peut être choisi parmi le groupe constitué de :

1.

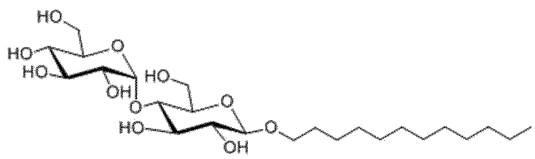

**n-Dodecyl-β-D-Maltopyranoside,**
n-Dodecyl-β-D-Maltoside
Lauryl Maltoside
Dodecyl 4-O-α-D-Glucopyranosyl-β-D-Glucopyranoside
DDM / LM

2.

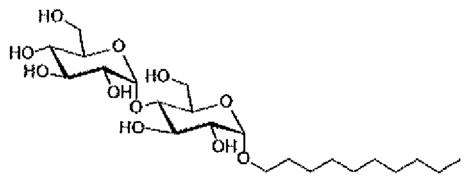

**n-Decyl-α-D-Maltopyranoside**
n-Decyl-α-D-Maltoside (High alpha)

Decyl Maltoside
DM

3.

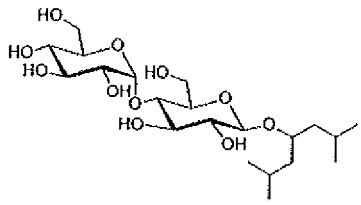

**2,6-Dimethyl-4-Heptyl-β-D-Maltopyranoside**

4.

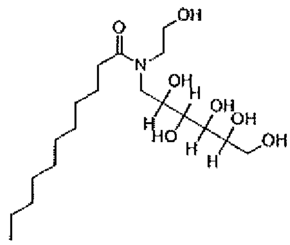

**HEGA-11**
Undecanoyl-N-Hydroxyethylglucamide

5.

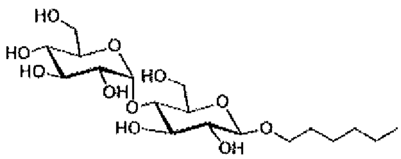

**n-Hexyl-β-D-Maltopyranoside**
n-Hexyl-β-D-Maltoside

6.

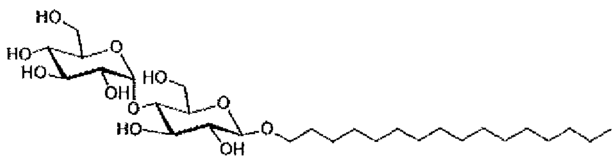

**n-Hexadecyl-β-D-Maltopyranoside**
n-Hexadecyl-β-D-Maltoside

7.

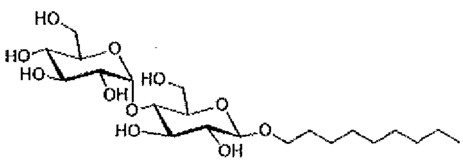

**n-Nonyl-β-D-Maltopyranoside**
n-Nonyl-β-D-Maltoside

8.

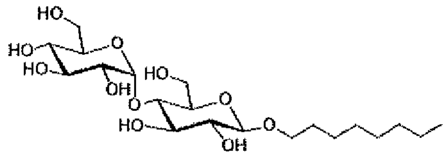

**n-Octyl-β-D-Maltopyranoside**
n-Octyl-β-D-Maltoside
OM

9.

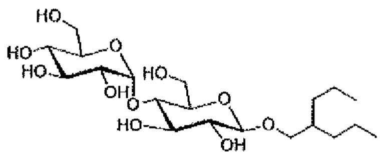

**2-Propyl-1-Pentyl-β-D-Maltopyranoside**

10.

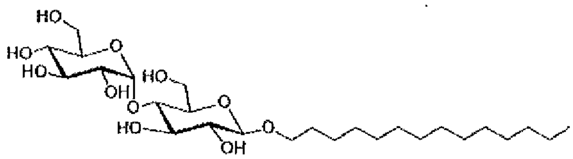

**n-Tetradecyl-β-D-Maltopyranoside**
n-Tetradecyl-β-D-Maltoside
Tetradecyl 4-O-α-D-Glucopyranosyl-β-D-Glucopyranoside

11.

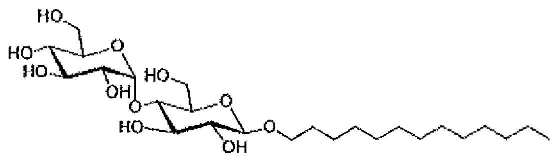

**n-Tridecyl-β-D-Maltopyranoside**
n-Tridecyl-β-D-Maltoside

12.

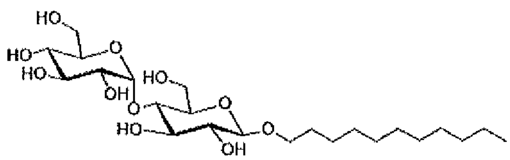

**n-Undecyl-β-D-Maltopyranoside**
n-Undecyl-β-D-Maltoside

UM

13.

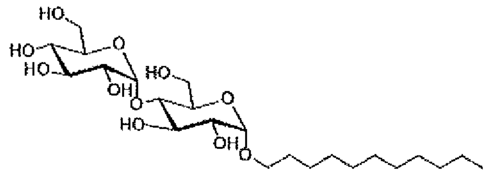

**n-Undecyl-α-D-Maltopyranoside**
n-Undecyl-α-D-Maltoside
UM

14.

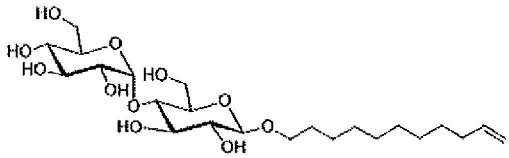

**ω-Undecylenyl-β-D-Maltopyranoside**
ω-Undecylenyl-β-D-Maltoside

15.

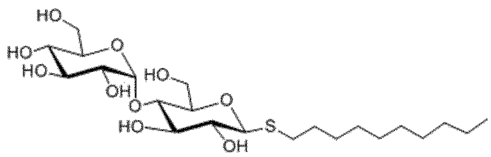

**n-Decyl-β-D-Thiomaltopyranoside**
n-Decyl-β-D-Thiomaltoside
DTM
Decyl Thiomaltoside

16.

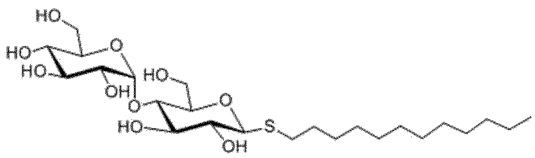

**n-Dodecyl-β-D-Thiomaltopyranoside**
n-Dodecyl-β-D-Thiomaltoside
LTM
Lauryl Thiomaltoside

17.

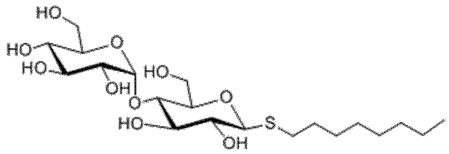

**n-Octyl-β-D-Thiomaltopyranoside**
n-Octyl-β-D-Thiomaltoside
NTM

18.

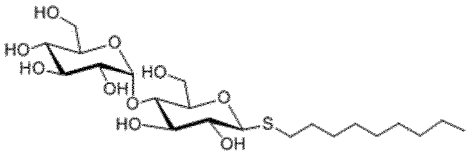

**n-Nonyl-β-D-Thiomaltopyranoside**
n-Nonyl-β-D-Thiomaltoside
NTM

19.

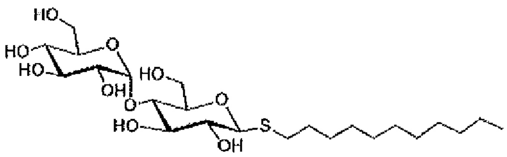

**n-Undecyl-β-D-Thiomaltopyranoside**
n-Undecyl-β-D-Thiomaltoside

20.

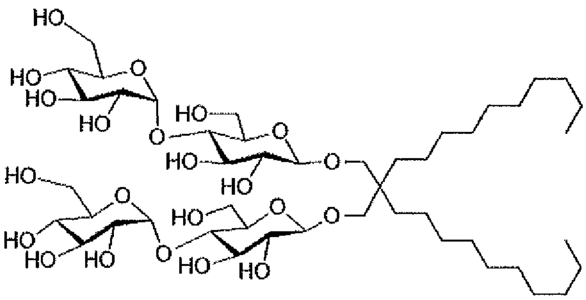

**Lauryl Maltose Neopentyl Glycol (MNG3)**
2,2-didecylpropane-1,3-bis-β-D-maltopyranoside
LMNG
MNG-DDM

21.

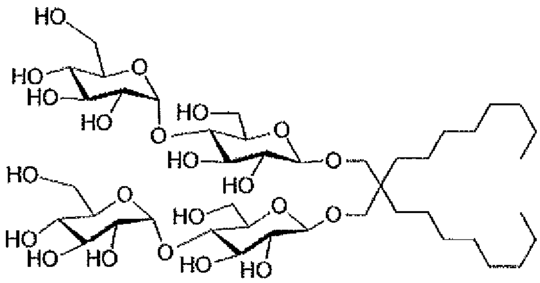

**Decyl Maltose Neopentyl Glycol**
2,2-dioctylpropane-1,3-bis-β-D-maltopyranoside
DMNG
MNG-DM

22.

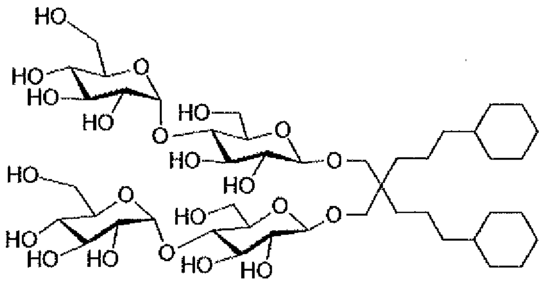

**CYMAL-5 Neopentyl Glycol**
(2,2-bis(3'-cyclohexylpropyl) propane-1,3-bis-β-D-maltopyranoside)
CYMAL-5 -NG

23.

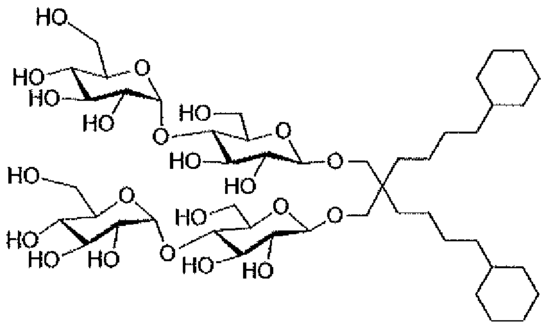

**CYMAL-6 Neopentyl Glycol**
(2,2-bis(3'-cyclohexylbutyl)propane-1,3-bis-β-D-maltopyranoside)
CYMAL-6-NG

24.

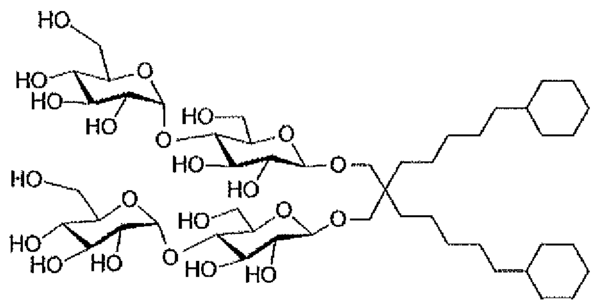

**CYMAL-7 Neopentyl Glycol**
(2,2-bis(3'-cyclohexylpentyl)propane-1,3-bis-β-D-maltopyranoside)
CYMAL-7-NG

25.

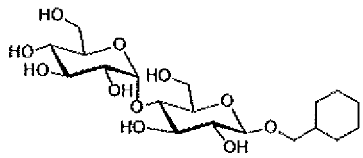

**CYMAL-1**
Cyclohexyl-Methyl-β-D-Maltoside1

26.

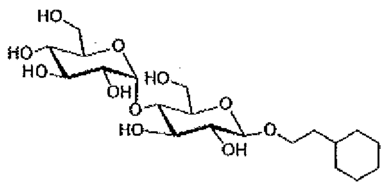

**CYMAL-2**
2-Cyclohexyl-1-Ethyl-β-D-Maltoside

27.

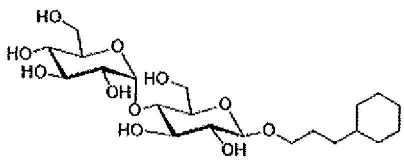

**CYMAL-3**
3-Cyclohexyl-1-Propyl-β-D-Maltoside

28.

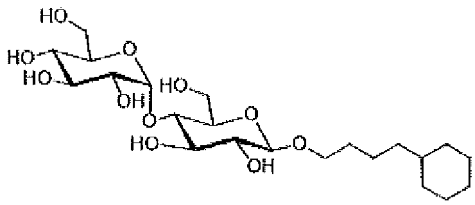

**CYMAL-4**
4-Cyclohexyl-1-Butyl-β-D-Maltoside

29.

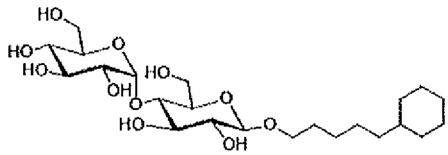

**CYMAL-5**
5-Cyclohexyl-1-Pentyl-β-D-Maltoside

30.

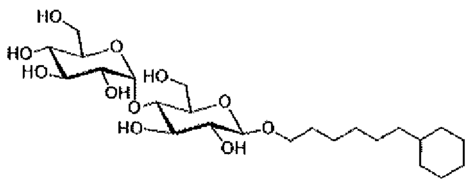

**CYMAL-6**
6-Cyclohexyl-1-Hexyl-β-D-Maltoside

31.

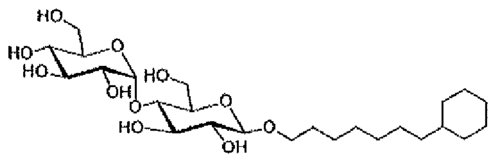

**CYMAL-7**
7-Cyclohexyl-1-Heptyl-β-D-Maltoside

32.

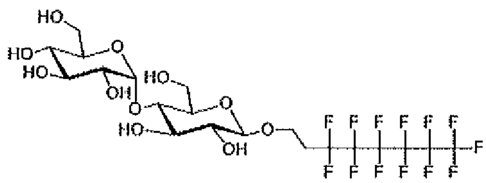

**Octyl Maltoside, Fluoré**
(1H, 1H, 2H, 2H-Perfluorooctyl)-β-D-Maltopyranoside
FOM

33.

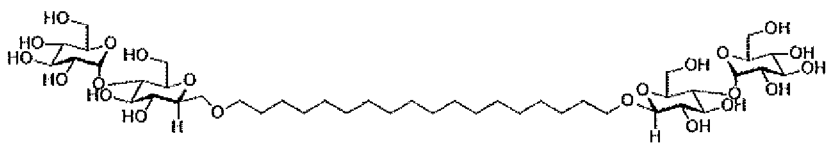

**BisMalt-18**
1,18-bis-(β-D-Maltopyranosyl) Octadecane

34.

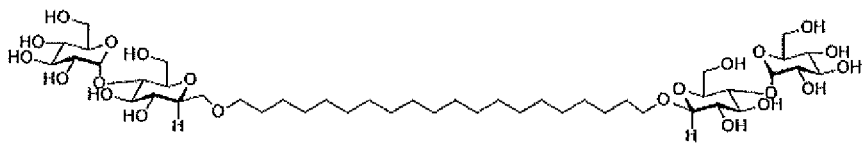

**BisMalt-20**
1,20-bis-(β-D-maltopyranosyl)-Docosane

35.

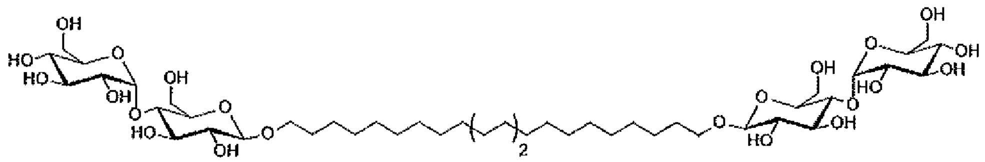

**BisMalt-22**
1,22-bis-(β-D-maltopyranosyl)-Docosane

36.

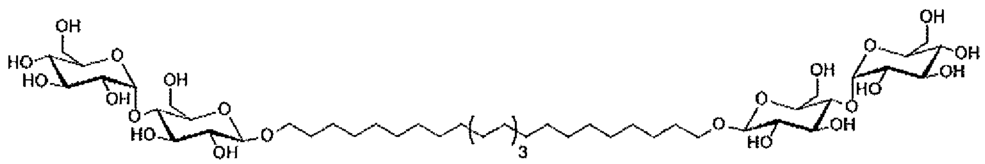

**BisMalt-24**
1,24-bis-(β-D-Maltopyranosyl)-Tetracosane

37.

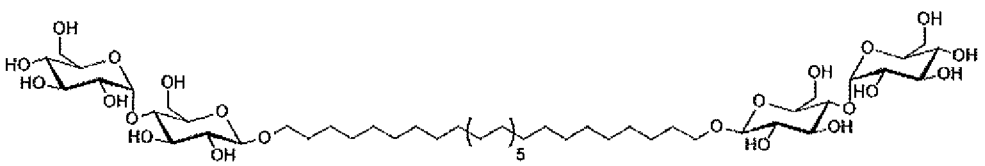

**BisMalt-28**
1,28-bis-(β-D-Maltopyranosyl)-Octacosane

38.

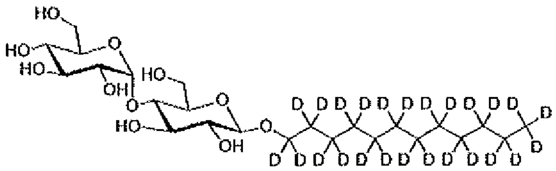

**n-Dodecyl-d25-β-D-Maltopyranoside**
n-Dodecyl-d25-β-D-Maltoside,
Lauryl Maltoside
DDM/LM

39.

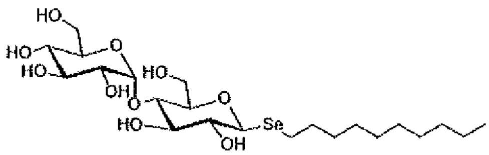

**Decyl-p-D-Selenomaltoside**
Decyl Selenomaltoside

40.

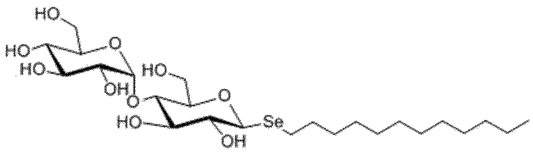

**Dodecyl-β-D-Selenomaltoside**
Selenium Maltoside

41.

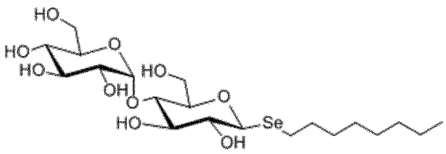

**Octyl-β-D-Selenomaltoside**
Selenium Maltoside

42.

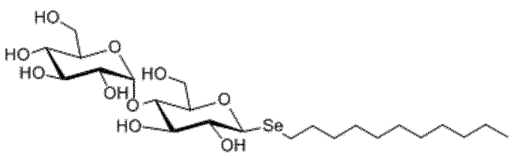

**Undecyl-β-D-Selenomaltoside**

Dans un procédé tel que défini ci-dessus, ledit au moins un détergent peut être le Lauryl Maltose Neopentyl Glycol (MNG3).

Dans un procédé tel que défini ci-dessus, ledit au moins un détergent peut être un mélange de détergents, en particulier de maltosides, de préférence un mélange CYMAL-7/DDM ou DDM/MNG3.

Dans un un procédé tel que défini ci-dessus, l'étape de solubilisation de ladite membrane cellulaire peut être réalisée à l'aide d'une solution comprenant 50 mM de Tris HCl à pH 7, 300 mM de NaCl et 5 mM de MNG3.

Un procédé tel que défini ci-dessus peut comprendre, à l'issue de l'étape de solubilisation, une étape de purification de ladite protéine Nox en suspension dans la solution appropriée.

Dans un procédé tel que défini ci-dessus, ledit au moins un détergent peut être utilisé à une concentration 0,01 mM à 0,2 mM, de préférence à 0,03 mM pendant ladite étape de purification.

Dans un procédé tel que défini ci-dessus, l'étape de purification de ladite protéine Nox peut être réalisée par chromatographie, de préférence à l'aide d'une résine ayant une forte affinité pour un motif d'acides aminés présent sur ladite protéine, ledit motif d'acides aminés étant de préférence une queue de poly-histidine.

Dans un procédé tel que défini ci-dessus, ladite résine peut être du sépharose.

De préférence, ladite résine est équilibrée avec la même solution que celle utilisée pour la solubilisation de la membrane, de préférence avec une solution comprenant 50 mM de Tris HCl à pH 7, 300 mM de NaCl et 5 mM de MNG3.

Dans un procédé tel que défini ci-dessus, lesdites cellules peuvent être des bactéries, de préférence choisies parmi le groupe consistant en *Streptococcus pneumoniae, Pseudomonas aeruginosa, Escherichia coli, Vibrio cholerae, Salmonella enterica* et *Bordetella pertussis.*

Un procédé tel que défini ci-dessus peut comprendre les étapes de :

- production de ladite protéine Nox dans des bactéries,
- lyse des bactéries pour obtenir un extrait cellulaire dans une solution appropriée,
- mise en contact de l'extrait cellulaire avec un détergent pour solubiliser les membranes cellulaires et obtenir ladite protéine Nox en suspension dans la solution appropriée,
- purification de ladite protéine Nox en suspension dans la solution appropriée.

Un procédé tel que défini ci-dessus, peut comprendre les étapes de :

- production de ladite protéine Nox dans des bactéries *E. coli,*
- lyse mécanique des bactéries par un micro-fluidizer pour obtenir un extrait cellulaire dans une solution appropriée,
- mise en contact de l'extrait cellulaire avec une solution comprenant 50 mM de Tris HCl à pH 7, 300 mM de NaCl et 5 mM de MNG3, pour solubiliser les membranes cellulaires et obtenir ladite protéine Nox en suspension dans la solution appropriée,
- purification de ladite protéine Nox en suspension dans la solution appropriée, par chromatographie d'affinité, avec un tampon de 50mM Tris pH7, 300mM NaCl, 0,03mM MNG3.

Un procédé tel que défini ci-dessus peut comprendre, après ladite étape de purification, une étape de chromatographie par exclusion de taille, notamment dans une colonne, de préférence à l'aide d'un tampon 50 mM Tris HCl pH 7, 300 mM NaCl et 20 à 100$\mu$M MNG3.

De préférence, le tampon utilisé pour l'étape de purification contient 30$\mu$M de MNG3.

Un procédé tel que défini ci-dessus peut comprendre, après ladite étape de chromatographie par exclusion de taille, une étape de déplétion du détergent.

L'étape de déplétion du détergent permet d'éliminer l'excédent de détergent de la protéine Nox dans une colonne d'affinité (ou colonne échangeuse d'ions). De préférence, 10 volumes de colonne sont utilisés pour laver le surplus de MNG3 et l'élution est effectuée avec un tampon 50mM Tris HCl pH 7, 300 mM NaCl et 20 $\mu$M MNG3.

Les protéines Nox utilisées selon l'invention peuvent être exprimées efficacement dans des bactéries, avec un rendement important de l'ordre du milligramme de protéine pure et fonctionnelle par litre de culture, cela permet une production à grande échelle et à un coût très faible.

Outre leur production en grande quantité et leur coût de production faible, les protéines Nox utilisées selon l'invention présentent de nombreux avantages pour le criblage de molécules d'intérêt thérapeutique. De manière non exhaustive, celles-ci sont par exemples activables avec seulement du NADPH, ou un analogue du NADPH, et sont pratiques à mettre en œuvre dans la mesure où il s'agit de système enzymatique à un seul composant.

Dans un mode de réalisation, l'invention concerne l'utilisation ci-dessus dans laquelle ladite protéine NOX est utilisée *in vitro.*

Dans un mode de réalisation, l'invention concerne l'utilisation ci-dessus dans laquelle ladite protéine NOX est utilisée *ex vivo.*

Dans un mode de réalisation, l'invention concerne l'utilisation ci-dessus dans laquelle ladite protéine NOX est

utilisée *in vivo* chez un animal non humain.

Dans un mode de réalisation particulier, ladite protéine Nox constitue une cible thérapeutique modèle.

Les protéines Nox utilisées selon l'invention constituent des protéines modèles particulièrement intéressantes, d'autant plus qu'il s'agit d'homologues aux protéines Nox eucaryotes, et notamment des protéines Nox humaines. En effet, la similarité entre SpNox et Nox2 est identique à la similarité entre Nox2 et Nox5. L'utilisation des protéines Nox de l'invention pour le pré-criblage ou le criblage de molécules d'intérêt thérapeutique permettrait donc d'augmenter la probabilité d'identifier de nouvelles drogues actives pour les Nox eucaryotes.

Dans un mode de réalisation particulier, dans l'utilisation de la protéine Nox définie précédemment, ladite protéine est mise en contact avec au moins une molécule d'intérêt thérapeutique.

Dans un mode de réalisation, dans l'utilisation de la protéine Nox définie précédemment, ladite protéine Nox est adsorbée sur un support, de préférence à partir d'une étiquette poly-histidine présente sur ladite protéine Nox.

Dans un mode de réalisation, dans l'utilisation de la protéine Nox définie précédemment, ledit support est une plaque, de préférence une plaque 96 puits.

Dans un mode de réalisation particulier, dans l'utilisation de la protéine Nox définie précédemment, l'activité NADPH oxydase de ladite protéine Nox et/ou la production de ROS est mesurée, en présence ou en absence de ladite au moins une molécule d'intérêt thérapeutique.

Dans un mode de réalisation, l'utilisation de la protéine Nox définie précédemment est pour identifier des molécules capables de moduler son activité NADPH oxydase.

Dans un mode de réalisation, l'utilisation de la protéine Nox définie précédemment est pour identifier des molécules capables de moduler son activité NADPH oxydase, ladite protéine Nox étant une protéine procaryote.

Dans un mode de réalisation, l'utilisation de la protéine Nox définie précédemment est pour identifier des molécules capables de moduler l'activité NADPH oxydase d'une autre protéine Nox.

Dans un mode de réalisation particulier, l'utilisation d'une protéine Nox procaryote telle que définie précédemment est pour identifier des molécules capables de moduler l'activité NADPH oxydase d'une protéine Nox eucaryote.

Dans un mode de réalisation particulier, l'utilisation de la protéine Nox définie précédemment est pour le criblage de molécules capables de moduler la production de ROS par ladite protéine Nox ou par une autre protéine Nox.

Dans un mode de réalisation particulier, l'utilisation de la protéine Nox définie précédemment est pour le criblage de molécules capables de réduire ou inhiber la production de ROS par ladite protéine Nox ou par une autre protéine Nox.

Dans un mode de réalisation particulier, l'utilisation de la protéine Nox définie précédemment est pour le criblage de molécules capables de stimuler la production de ROS par ladite protéine Nox ou par une autre protéine Nox.

Dans un mode de réalisation particulier, l'utilisation de la protéine Nox définie précédemment est pour le criblage de molécules susceptibles de présenter des propriétés antibiotiques.

L'identification de molécules capables de stimuler la production de ROS par une Nox bactérienne peut également permettre la potentialisation d'antibiotiques connus. En effet, la production de ROS endogène dans la bactérie peut avoir pour conséquence de rendre sensible à des antibiotiques des bactéries résistantes.

De telles molécules augmentant la production de ROS par les NOX peuvent ainsi être utilisées en combinaison avec un antibiotique pour le traitement de certaines infections bactériennes.

En d'autres termes, une molécule activatrice d'une protéine NOX procaryote est susceptible d'entrainer une production de ROS lorsqu'elle est administrée à une bactérie et, par conséquent, est susceptible de constituer une molécule anti-bactérienne efficace.

Un autre aspect de l'invention concerne l'utilisation *in vitro, ex vivo* ou *in vivo :*

- d'un acide nucléique codant une protéine Nox telle que définie précédemment,
- d'un vecteur d'expression contenant ledit acide nucléique, ou
- d'une cellule contenant ledit vecteur d'expression,

comme outil de recherche pour le criblage de molécules présentant un intérêt thérapeutique, ledit acide nucléique, ledit vecteur d'expression ou ladite cellule n'étant pas utilisés *in vivo* chez l'homme.

Dans un mode de réalisation, l'invention concerne l'utilisation ci-dessus dans laquelle ledit acide nucléique, ledit vecteur d'expression ou ladite cellule est utilisé *in vitro.*

Dans un mode de réalisation, l'invention concerne l'utilisation ci-dessus dans laquelle ledit acide nucléique, ledit vecteur d'expression ou ladite cellule est utilisé *ex vivo.*

Dans un mode de réalisation, l'invention concerne l'utilisation ci-dessus dans laquelle ledit acide nucléique, ledit vecteur d'expression ou ladite cellule est utilisé *in vivo* chez un animal non humain.

Un procédé d'identification, à partir d'une banque de molécules à tester, d'au moins une molécule capable de modifier la production de ROS par une protéine Nox eucaryote, comprend :

- une étape a) où lesdites molécules à tester sont criblées, par mise en contact avec une protéine Nox procaryote,

pour sélectionner les molécules capables de modifier la production de ROS par la protéine Nox procaryote, puis

- une étape b) où lesdites molécules capables de modifier la production de ROS par la protéine Nox procaryote sont mises en contact avec la protéine Nox eucaryote, pour identifier au moins une molécule capable de modifier la production de ROS par la protéine Nox eucaryote.

Les protéines Nox procaryotes pouvant être produites à grande échelle et à faible coût, il est intéressant de les utiliser pour tester et sélectionner préalablement des molécules susceptibles d'agir sur les protéines Nox eucaryotes, ces dernières étant plus difficiles à produire en quantités suffisantes.
La production de ROS par ladite protéine Nox procaryote à l'étape a), et/ou par ladite protéine Nox eucaryote à l'étape b), peut être déterminée en effectuant une mesure d'absorbance par spectrophotométrie, et en particulier par un test colorimétrique, par chimioluminescence ou par fluorescence.
Les étapes a) et b) peuvent être réalisées dans des plaques, de préférence des plaques 96 puits.
L'étape a) peut être réalisée en plaque, de préférence des plaques 96 puits, et l'étape b) peut être réalisée en cuve de spectrophotométrie.
L'étape a) peut être réalisée en cuve de spectrophotométrie et l'étape b) peut être réalisée en plaque, de préférence des plaques 96 puits.
Le motif :
[P/A/S/E/F]-H-[P/S/A]-F-[T/S]-[L/I/M/V] (SEQ ID NO : 2)
peut être utilisé pour identifier une séquence d'acides aminés susceptible de coder une protéine Nox.
Le motif :
G-[I/S/V/L]-G-[V/I/A/F]-[T/A/S]-[P/Y/T/A] (SEQ ID NO : 4)

peut être utilisé pour identifier une séquence d'acides aminés susceptible de coder une protéine Nox.
Le motif :
[P/A/S/E/F]-H-[P/S/A]-F-[T/S]-[L/I/M/V] (SEQ ID NO : 2)
et le motif :
G-[I/S/V/L]-G-[V/I/A/F]-[T/A/S]-[P/Y/T/A] (SEQ ID NO : 4)
peuvent être utilisés pour identifier une séquence d'acides aminés susceptible de coder une protéine Nox.
Un procédé d'identification d'une protéine Nox comprend :

a) une étape de détection dans une séquence d'acides aminés d'un motif NADPH comprenant la séquence suivante : G-[I/S/V/L]-G-[V/I/A/F]-[T/A/S]-[P/Y/T/A] (SEQ ID NO : 4), et
b) une étape de détection dans ladite séquence d'acides aminés d'un motif FAD comprenant la séquence suivante : H-[P/S/A]-F-[T/S]-[L/I/M/V] (SEQ ID NO : 6), et
où si ladite séquence d'acides aminés comprend ledit motif NADPH de l'étape a) et le dit motif FAD de l'étape b), alors ladite séquence d'acides aminés est susceptible de coder une protéine Nox.

Un procédé d'identification *in silico* d'une protéine Nox comprend :

a) une étape de détection dans une séquence d'acides aminés d'un motif NADPH comprenant la séquence suivante : G-[I/S/V/L]-G-[V/I/A/F]-[T/A/S]-[P/Y/T/A] (SEQ ID NO : 4), et
b) une étape de détection dans ladite séquence d'acides aminés d'un motif FAD comprenant la séquence suivante : H-[P/S/A]-F-[T/S]-[L/I/M/V] (SEQ ID NO : 6), et
où si ladite séquence d'acides aminés comprend ledit motif NADPH de l'étape a) et le dit motif FAD de l'étape b), alors ladite séquence d'acides aminés est susceptible de coder une protéine Nox.

Un procédé d'identification tel que défini ci-dessus, peut comprendre en plus :

c) une étape de détection dans ladite séquence d'acides aminés de 3 à 7 régions susceptibles de former des hélices transmembranaires, notamment à l'aide du logiciel TMHMM, et
d) une étape de détection dans ladite séquence d'acides aminés d'au moins 2 motifs bis-histidyl séparés par au moins 20 acides aminés, chacun des motifs bis-histidyl étant constitué de 2 résidus histidine séparés par 12, 13 ou 14 résidus d'acides aminés, et

où si ladite séquence d'acides aminés comprend lesdites 3 à 7 régions susceptibles de former des hélices transmembranaires et lesdits au moins 2 motifs bis-histidyl, alors ladite séquence d'acides aminés est susceptible de coder une protéine Nox.
Dans un procédé d'identification tel que défini ci-dessus ledit motif FAD de l'étape b) peut comprendre la

séquence :
[P/A/S/E/F]-H-[P/S/A]-F-[T/S]-[L/I/M/V] (SEQ ID NO : 2).

Un procédé d'identification tel que défini ci-dessus, peut comprendre une étape de détection dans ladite séquence d'acides aminés d'un motif supplémentaire S1:
[G/S/A]-[Q/D]-F-[A/V/T/L/F]-[F/Y/W/L/R]-[L/V/I/F/W] (SEQ ID NO : 1), et
où si ladite séquence d'acides aminés comprend ledit motif supplémentaire S1, alors ladite séquence d'acides aminés est susceptible de coder une protéine Nox.

Un procédé d'identification tel que défini ci-dessus, peut comprendre une étape de détection dans ladite séquence d'acides aminés d'un motif supplémentaire S2 :
[K/R]-X-X-G-[D/G]-X-[T/S] (SEQ ID NO : 3), et
où si ladite séquence d'acides aminés comprend ledit motif supplémentaire S2, alors ladite séquence d'acides aminés est susceptible de coder une protéine Nox.

Dans un procédé d'identification tel que défini ci-dessus, la dite séquence d'acides aminés peut être codée par une séquence nucléotidique présente sur un génome bactérien.

Un procédé tel que défini précédemmentpeut comprendre :

- une étape a) de détection dans une séquence d'acides aminés d'un motif NADPH comprenant la séquence suivante : G-[I/S/V/L]-G-[V/I/A/F]-[T/A/S]-[P/Y/T/A] (SEQ ID NO : 4), et
- une étape b) de détection dans ladite séquence d'acides aminés d'un motif FAD comprenant la séquence suivante : H-[P/S/A]-F-[T/S]-[L/I/M/V] (SEQ ID NO : 6), et
- une étape c) de détection dans ladite séquence d'acides aminés de 3 à 7 régions susceptibles de former des hélices transmembranaires, notamment à l'aide du logiciel TMHMM, et
- une étape d) de détection dans ladite séquence d'acides aminés d'au moins 2 motifs bis-histidyl, chacun des motifs bis-histidyl étant constitué de 2 résidus histidine séparés par 12, 13 ou 14 résidus d'acides aminés, lesdits motifs bis-histidyl étant séparés par 20 acides aminés,

où si ladite séquence d'acides aminés comprend ledit motif NADPH de l'étape a), le dit motif FAD de l'étape b), lesdites 3 à 7 régions susceptibles de former des hélices transmembranaires de l'étape c) et lesdits au moins 2 motifs bis-histidyl de l'étape d),
alors ladite séquence d'acides aminés est susceptible de coder une protéine NADPH oxydase.

Un procédé d'identification d'une protéine Nox procaryote comprend :

- une étape a) de détection dans une séquence d'acides aminés d'un motif NADPH comprenant la séquence suivante : G-[I/S/V/L]-G-[V/I/A/F]-[T/A/S]-[P/Y/T/A] (SEQ ID NO : 4), et
- une étape b) de détection dans ladite séquence d'acides aminés d'un motif FAD comprenant la séquence suivante : [P/A/S/E/F]-H-[P/S/A]-F-[T/S]-[L/I/M/V] (SEQ ID NO : 2), et
- une étape c) de détection dans ladite séquence d'acides aminés de 3 à 7 régions susceptibles de former des hélices transmembranaires, notamment à l'aide du logiciel TMHMM, et
- une étape d) de détection dans ladite séquence d'acides aminés d'au moins 2 motifs bis-histidyl, chacun des motifs bis-histidyl étant constitué de 2 résidus histidine séparés par 12, 13 ou 14 résidus d'acides aminés, lesdits motifs bis-histidyl étant séparés par 20 acides aminés, et
- une étape e) de détection dans ladite séquence d'acides aminés d'un supplémentaire S1 : [G/S/A]-[Q/D]-F-[A/V/T/L/F]-[F/Y/W/L/R]-[L/V/I/F/W] (SEQ ID NO : 1), et
- une étape f) de détection dans ladite séquence d'acides aminés d'un deuxième motif supplémentaire S2 : [K/R]-X-X-G-[D/G]-X-[T/S] (SEQ ID NO : 3)
  où si ladite séquence d'acides aminés comprend ledit motif NADPH de l'étape a), le dit motif FAD de l'étape b), lesdites 3 à 7 régions susceptibles de former des hélices transmembranaires de l'étape c), lesdits au moins 2 motifs bis-histidyl de l'étape d), le motif supplémentaire S1 de l'étape e) et le motif supplémentaire S2 de l'étape f), alors ladite séquence d'acides aminés est susceptible de coder une protéine Nox procaryote.

Un procédé d'identification *in silico* d'une protéine Nox procaryote comprend :

- une étape a) de détection dans une séquence d'acides aminés d'un motif NADPH comprenant la séquence suivante : G-[I/S/V/L]-G-[V/I/A/F]-[T/A/S]-[P/Y/T/A] (SEQ ID NO : 4), et
- une étape b) de détection dans ladite séquence d'acides aminés d'un motif FAD comprenant la séquence suivante : [P/A/S/E/F]-H-[P/S/A]-F-[T/S]-[L/I/M/V] (SEQ ID NO : 2), et
- une étape c) de détection dans ladite séquence d'acides aminés de 3 à 7 régions susceptibles de former des hélices transmembranaires, notamment à l'aide du logiciel TMHMM, et - une étape d) de détection dans ladite séquence

d'acides aminés d'au moins 2 motifs bis-histidyl, chacun des motifs bis-histidyl étant constitué de 2 résidus histidine séparés par 12, 13 ou 14 résidus d'acides aminés, lesdits motifs bis-histidyl étant séparés par 20 acides aminés, et

- une étape e) de détection dans ladite séquence d'acides aminés d'un supplémentaire S1 : [G/S/A]-[Q/D]-F-[A/V/T/L/F]-[F/Y/W/L/R]-[L/V/I/F/W] (SEQ ID NO : 1), et
- une étape f) de détection dans ladite séquence d'acides aminés d'un deuxième motif supplémentaire S2 : [K/R]-X-X-G-[D/G]-X-[T/S] (SEQ ID NO : 3)
  où si ladite séquence d'acides aminés comprend ledit motif NADPH de l'étape a), le dit motif FAD de l'étape b), lesdites 3 à 7 régions susceptibles de former des hélices transmembranaires de l'étape c), lesdits au moins 2 motifs bis-histidyl de l'étape d), le motif supplémentaire S1 de l'étape e) et le motif supplémentaire S2 de l'étape f), alors ladite séquence d'acides aminés est susceptible de coder une protéine Nox procaryote.

Un détergent de la famille des maltosides peut être utilisé pour la solubilisation de protéines Nox.

Dans l'utilisation d'un détergent de la famille des maltosides pour la solubilisation de protéines Nox, ledit détergent est de préférence choisi parmi le groupe des dodecyl-maltosides.

Dans l'utilisation d'un détergent de la famille des maltosides pour la solubilisation de protéines Nox, ledit détergent est de préférence choisi parmi le groupe constitué de :

- n-Dodecyl-β-D-Maltopyranoside,
- n-Decyl-α-D-Maltopyranoside,
- 2,6-Dimethyl-4-Heptyl-β-D-Maltopyranoside,
- HEGA-11,
- n-Hexyl-β-D-Maltopyranoside,
- n-Hexadecyl-β-D-Maltopyranoside,
- n-Nonyl-β-D-Maltopyranoside,
- n-Octyl-β-D-Maltopyranoside,
- 2-Propyl-1-Pentyl-β-D-Maltopyranoside,
- n-Tetradecyl-β-D-Maltopyranoside,
- n-Tridecyl-β-D-Maltopyranoside,
- n-Undecyl-β-D-Maltopyranoside,
- n-Undecyl-α-D-Maltopyranoside,
- ω-Undecylenyl-β-D-Maltopyranoside,
- n-Decyl-β-D-Thiomaltopyranoside,
- n-Dodecyl-β-D-Thiomaltopyranoside,
- n-Octyl-β-D-Thiomaltopyranoside,
- n-Nonyl-β-D-Thiomaltopyranoside,
- n-Undecyl-β-D-Thiomaltopyranoside,
- Lauryl Maltose Neopentyl Glycol (MNG3),
- Decyl Maltose Neopentyl Glycol,
- CYMAL-5 Neopentyl Glycol,
- CYMAL-6 Neopentyl Glycol,
- CYMAL-7 Neopentyl Glycol,
- CYMAL-1,
- CYMAL-2,
- CYMAL-3,
- CYMAL-4,
- CYMAL-5,
- CYMAL-6,
- CYMAL-7,
- Octyl Maltoside, Fluoré,
- BisMalt-18,
- BisMalt-20,
- BisMalt-22,
- BisMalt-24,
- BisMalt-28,
- n-Dodecyl-d25-β-D-Maltopyranoside,
- Decyl-β-D-Selenomaltoside,
- Dodecyl-β-D-Selenomaltoside,
- Octyl-β-D-Selenomaltoside, et

- Undecyl-β-D-Selenomaltoside.

## LEGENDES DES FIGURES

**Figure 1.** Arbre phylogénétique des protéines Nox.

**Figure 2**. Représentation schématique des protéines Nox2 (Nox humaine) (A), SpNox (*Streptococcus pneumoniae*) (B), PaNox (*Pseudomonas aeruginosa*) et EcNox (*Escherichia coli*) (D).

**Figure 3.** Analyse par SDS-PAGE 10% acrylamide en coloration au bleu de Coomassie (à gauche) et Western-blot immuno-révélé par un anticorps dirigé contre l'étiquette poly-histidine de spNOX (à droite). T0, tl, t2 et t3 : Extraits totaux avant induction, après 1h, 2h et 3h. + : contrôle positif.

**Figure 4.** Test d'activité sur 10 μg de protéines membranaires totales. Le transfert des électrons du NADPH, substrat de SpNox (protéine Nox de *Streptococcus pneumoniae*), au FAD, son cofacteur, puis aux hèmes et à l'oxygène, comme accepteur final, permet de produire des espèces réactives de l'oxygène (ROS). Ces ROS réduiront le cytochrome c qui absorbera alors à 550 nm.

**Figure 5.** Mesure de spectrophotométrie entre 250 et 700 nm. La présence des hèmes dans la protéine induit une signature caractéristique avec un pic à 410 nm. La centrifugation à très haute vitesse permet d'éliminer des agrégats présents dans l'échantillon. Le pic à 280 nm diminue mais pas celui à 410 nm.

**Figure 6. A)** Suivi de la purification de SpNox sur colonnes d'affinité et gel filtration par SDS-PAGE 10% acrylamide en coloration au bleu de Coomassie. Std : standard de poids moléculaire (KDa), M : membrane, FT : Flow throught, L : lavage, E : Elution, A : Gel filtration, C1: 2 μg de SpNox, C2 : 3 μg de SpNox, C3 : 6 μg de SpNox. **B)** MALDI-TOF MS, spectre de SpNox purifiée. L'échantillon a été concentré à 3 mg/ml et dilué dans une matrice SA.

**Figure 7. A)** Activité de 1 μg de SpNox. **B)** Spectre entre 250 et 700 nm de la protéine pure.

**Figure 8. A)** Représentation du MNG3 avec dans le cadre, les têtes glucidiques. **B)** Interférogramme de SpNox concentrée à 3 mg/mL avant et après déplétion du MNG3. Le signal des têtes maltose est entre 980 et 1150 $cm^{-1}$.

**Figure 9.** Western blot immunorévélé par un anticorps dirigé contre l'étiquette poly-histidine des protéines Nox produites. AmNox (*Acaryochloris marina*), EcNox (*Escherichia coli*), PaNox (*Pseudomonas aeruginosa*) et SpNox (*Streptococcus pneunomiae*).

**Figure 10.** Analyse de l'activité NADPH oxydase de *Sp*NOX par colorimétrie, en présence de DPI et d'acide arachidonique (AA). La présence du DPI inhibe la réaction car la coloration est moins marquée, alors que l'acide arachidonique stimule la production de superoxide et donc la réduction des sels de tétrazolium.

**Figure 11.** Mesure de l'activité NADPH oxydase de *Sp*Nox en présence de DPI. L'axe des ordonnées indique l'absorbance de l'échantillon à 560 nm. L'axe des abscisses indique la concentration (0,005 - 0,05 - 0,5 - 5 - 50 μM) en DPI dans l'échantillon.

**Figure 12.** Mesure de l'activité NADPH oxydase de *Sp*Nox en présence de catéchine. L'axe des ordonnées indique l'absorbance de l'échantillon à 560 nm. L'axe des abscisses indique la concentration (0,01 - 0,1 - 1 - 10 - 100 μM) μM en catéchine dans l'échantillon.

**Figure 13.** Mesure de l'activité NADPH oxydase de *Sp*Nox en présence d'épicatéchine gallate. L'axe des ordonnées indique l'absorbance de l'échantillon à 560 nm. L'axe des abscisses indique la concentration (0,01 - 0,1 - 1 - 10 - 100 μM) en épicatéchine gallate dans l'échantillon.

**Figure 14.** Mesure de l'activité NADPH oxydase de *Sp*Nox en présence d'ebsélène. L'axe des ordonnées indique l'absorbance de l'échantillon à 560 nm. L'axe des abscisses indique la concentration (0,01 - 0,1 - 1 - 10 - 100 μM) en ebsélène dans l'échantillon.

**Figure 15.** Mesure de l'activité NADPH oxydase de *Sp*Nox en présence de VAS2870. L'axe des ordonnées indique l'absorbance de l'échantillon à 560 nm. L'axe des abscisses indique la concentration (0,01 - 0,1 - 1 - 10 - 100 μM) en VAS2870 dans l'échantillon.

**Figure 16.** Mesure de l'activité NADPH oxydase de *Sp*Nox en présence de célastrol. L'axe des ordonnées indique l'absorbance de l'échantillon à 560 nm. L'axe des abscisses indique la concentration (0,01 - 0,1 - 1 - 10 - 100 μM) en célastrol dans l'échantillon.

**Figure 17.** Mesure de l'activité NADPH oxydase de *Sp*Nox en présence de Thr101. L'axe des ordonnées indique l'absorbance de l'échantillon à 560 nm. L'axe des abscisses indique la concentration (0,01 - 0,1 - 1 - 10 - 100 μM) en Thr101 dans l'échantillon.

**Figure 18**. Mesure de l'activité NADPH oxydase de *Sp*Nox en présence d'acide arachidonique. L'axe des ordonnées indique l'absorbance de l'échantillon à 560 nm. L'axe des abscisses indique la concentration (0,01 - 0,1 - 1 - 10 - 100 μM) en acide arachidonique dans l'échantillon.

## EXEMPLES

### Exemple 1 : Identification des protéines Nox à partir des séquences dans les bases de données

Le programme utilisé pour identifier les séquences de Nox à partir des génomes bactériens a été écrit en Python 3.0. Ce programme repose sur l'utilisation de 3 filtres complémentaires.

Les 2 premiers filtres sélectionnent les séquences contenant les motifs G[I/S/V/L]G[V/I/A/F][T/A/S][P/Y/T/A] (SEQ ID NO : 4) (domaine de fixation du NADPH) et H[P/S/A]F[T/S][L/I/M/V] (SEQ ID NO : 6) (domaine de fixation du FAD), définis à partir des séquences des 48 protéines les plus similaires par rapport à la protéine Nox2 humaine (Uniprot code : P04839). Les séquences des 48 protéines ont été identifiées en effectuant une recherche BlastP sur les bases de données publiques.

Le troisième filtre sélectionne les séquences qui contiennent les 4 résidus histidine responsables de coordonner les 2 parties d'hème du motif cytochrome b. Il est ainsi recherché 2 motifs bis-histidyl séparés par au moins 20 acides aminés (soit la taille d'une hélice transmembranaire), chacun des motifs bis-histidyl étant constitué de 2 résidus

Les 2 premiers filtres sélectionnent les séquences contenant les motifs G[I/S/V/L]G[V/I/A/F][T/A/S][P/Y/T/A] (SEQ ID NO : 4) (domaine de fixation du NADPH) et H[P/S/A]F[T/S][L/I/M/V] (SEQ ID NO : 6) (domaine de fixation du FAD), définis à partir des séquences des 48 protéines les plus similaires par rapport à la protéine Nox2 humaine (Uniprot code : P04839). Les séquences des 48 protéines ont été identifiées en effectuant une recherche BlastP sur les bases de données publiques.

Le troisième filtre sélectionne les séquences qui contiennent les 4 résidus histidine responsables de coordonner les 2 parties d'hème du motif cytochrome b. Il est ainsi recherché 2 motifs bis-histidyl séparés par au moins 20 acides aminés (soit la taille d'une hélice transmembranaire), chacun des motifs bis-histidyl étant constitué de 2 résidus histidine séparés par 12, 13 ou 14 résidus d'acides aminés. Le logiciel TMHMM a été utilisé pour localiser les hélices α transmembranaires. Les séquences avec plus de 7 hélices α prédites (c'est-à-dire le nombre d'hélices trouvées dans Duox1-2) n'ont pas été retenues, tandis que les séquences avec au moins 3 hélices α prédites ont été sélectionnées pour tenir compte des éventuelles erreurs de prédiction.

Les alignements multiples des séquences de protéines Nox identifiées ont été réalisés à l'aide du logiciel Clustal Omega en effectuant 2 itérations.

Un arbre phylogénétique a été construit avec le logiciel PhyML 3.0 à partir des séquences de 150 protéines Nox putatives (Figure 1).

### Exemple 2 : Préparation de la protéine Nox identifiée chez *Streptococcus pneumoniae* (*Sp*NOX)

Le gène codant pour la protéine SpNox (code UniProt #Q8CZ28) de *Streptococcus pneumoniae* R6 (identifiant taxonomique #171101) a été synthétisé et optimisé pour faire faire de l'expression hétérologue chez *Escherichia coli.* Des bases codant pour une étiquette poly-histidine ainsi qu'un site de digestion par la thrombine ont été ajoutés du côté 5' de l'ADN complémentaire codant SpNox.

La traduction du gène introduit 18 acides aminés du côté amino-terminal de la protéine avant digestion par la thrombine et seulement 4 acides aminés (GSRS) après. Le gène ainsi modifié a été cloné dans le vecteur pUC57.

Pour produire la protéine en quantités importantes, le gène modifié a été introduit dans le vecteur d'expression pQlinkN entre les sites de restriction NotI et BamHI sous le contrôle d'un promoteur tac, inductible à l'IPTG (isopropyl β-D-1-thiogalactopyranoside), et ce vecteur porte également un gène de résistance à l'ampicilline. Le vecteur a été

introduit par transformation par choc thermique dans une souche d'*Escherichia coli* BL21 (DE3) pLysS (phénotype : $F^-$ *ompT hsdSB* ($r_B^- m_B^-$) *gal dcm* (DE3) pLysS ($Cam^R$)) résistante au chloramphénicol. Les bactéries ont ensuite été immédiatement mises en culture 1h à 37°C dans un milieu LB (Luria Bertani) sans pression de sélection. Une fraction de cette culture a ensuite été étalée sur une boîte de pétri et incubée à 37°c toute une nuit.

A partir d'une seule colonie, une culture a été démarrée en milieu liquide en présence de chloramphénicol (34 μg/ml) et d'ampicilline (100 μg/ml) à 37°C, sous agitation pendant toute une nuit. L'inoculation de la culture a été faite à partir de la pré-culture selon une dilution au 1/20ème du volume final. La culture a été placée à 37°C et dans un agitateur orbital avec une vitesse de 180rpm. La densité optique du milieu a été suivie à 600 nm jusqu'à ce que la valeur de 1,5 soit atteinte. La surexpression de la protéine *Sp*NOX a été induite par l'ajout dans le milieu d'une concentration de 0,1 mM d'IPTG pendant 4h et à 210 rpm. Au terme de la culture, les bactéries ont été récoltées par centrifugation à basse vitesse (7000g, 30 min, 4°C) et entreposées à -80°C. Le niveau d'expression de la protéine SpNOX a ensuite été contrôlé en réalisant une analyse SDS-PAGE et confirmé par Western blot (Figure 3).

Le culot bactérien a été repris dans un tampon 50 mM Tris HCl à pH 7, 250 mM NaCl et 10% glycérol auquel un cocktail d'anti-protéases (complete ULTRA Tablets, EDTA-free, Roche Applied Science) a été ajouté, ainsi que de la DNase à une concentration de 10 μg/ml (Sigma-Aldrich). Les cellules ont été lysées mécaniquement grâce à un micro-fluidizer (M-1105, Microfluidics, USA) à 1,400 Psi en 5 passages successifs. Un contrôle visuel de la viscosité de l'échantillon est important à cette étape. Les bactéries non lysées, les débris cellulaires et les corps d'inclusion ont été séparés par centrifugation à basse vitesse (8000g, 20 min, 4°C). Le surnageant a été centrifugé à très haute vitesse pendant 1h à 250000g et à 4°C. Le culot a été repris dans un tampon 50 mM Tris pH7 et la concentration en protéine totale est déterminée par la technique BCA (bicinchoninic acid assay) et l'absorbance est mesurée à 562 nm selon le protocole mis à disposition par le fournisseur (Sigma-Aldrich). L'échantillon est stocké à -80°C à une concentration de 20 mg/ml de protéine totale.

L'activité de SpNox a été testée à cette étape afin de contrôler son intégrité après l'étape de lyse (Figure 4). Cette expérience est basée sur le suivi de la réduction du cytochrome c par les ions superoxyde produits par SpNox, selon le protocole disponible dans la publication Green *et al.* (Green TR, Wu DE. Detection of NADPH diaphorase activity associated with human neutrophil NADPH-O2 oxidoreductase activity. FEBS Lett. 1985 Jan 1; 179(1):82-6).

Les membranes ont été solubilisées dans un tampon 50 mM Tris HCl à pH 7, 300 mM NaCl et 5 mM de MNG3 (lauryl Maltose NeoPentyl Glycol - NG310-MNG - Affymetrix) à une concentration finale de 0,5 mg/ml de protéine totale. De la résine Ni-NTA *Sepharose High performance* (GE Healthcare Life Sciences), initialement équilibrée dans le même tampon de solubilisation, a été ajoutée à l'échantillon à une concentration de 1 ml de résine pour 15 mg de protéine. L'ensemble a été incubé sous agitation à 4°C pendant une nuit.

La résine chargée de la protéine a été déposée sur une colonne à gravité jusqu'à ce que le filtrat se soit complètement écoulé. Plusieurs lavages ont été effectués avec un tampon de 50 mM Tris HCl pH7, 300 mM NaCl, 50 μM MNG3 et 50 mM imidazole jusqu'à ce que l'absorbance à 280 nm soit stabilisée. Un palier à 300 mM d'imidazole permet d'éluer la protéine en étalant le retentât sur 3 à 5 ml.

La signature spectrale des différentes fractions a été évaluée entre 200 et 700 nm et la purification a été suivie par SDS-PAGE. Les fractions les plus pures ont été rassemblées et concentrées par filtration sur membrane avec une limite de taille de 30kDa (Amicon® Ultra-4 - Millipore). L'échantillon ainsi préparé a été centrifugé à haute vitesse, 150000g, pendant 20 minutes et à 4°C. Le rendement et la qualité de la purification ont été évalués par spectroscopie (Figure 5) et SDS-PAGE (Figure 6).

Afin de parfaire la purification de SpNox, une étape de chromatographie par exclusion de taille est nécessaire. Pour cela, une colonne *Superdex 200 10/300 GL* (GE Healthcare Life Sciences) a été utilisée avec une phase mobile constituée d'un tampon 50 mM Tris HCl pH 7, 300 mM NaCl et 50 μM MNG3. L'élution est suivie par absorbance à 280 nm grâce à un système de type ÄKTA FPLC (GE Healthcare Life Sciences). La pureté de la protéine a été analysée par SDS-PAGE et spectrométrie de masse (Figure 6).

Son activité a été testée (Figure 7). Ces données ont permis de déterminer un facteur de purification proche de 17 et un rendement de purification de 88%.

Une colonne d'affinité *CM Sepharose Fast Flow* (GE Healthcare Life Sciences) a été utilisée afin de dépléter l'excédent de détergent de la protéine *Sp*Nox. L'échantillon a été dilué afin de ramener la concentration en NaCl sous le seuil des 15 mM grâce à un tampon 50 mM Tris HCl pH7 et 20 μM MNG3. La quantité de résine utilisée est dépendante de la quantité de protéine disponible avec un ratio de 0,5 ml de résine pour 3 mg de SpNox. Dix volumes de colonne ont été utilisés pour laver le surplus de MNG3. 65 à 70% de la protéine a été récupérée.

L'échantillon a été contrôlé par spectroscopie FT-IR et ultracentrifugation analytique (Figure 7).

L'échantillon obtenu peut être concentré et/ou stocké.

**Exemple 3 : Protocole de mesure de l'activité NADPH oxydase de membranes de BL21 (DE3) pLys surexprimant *Sp*Nox par colorimétrie**

L'activité oxydase est déterminée par mesure de la cinétique de réduction du cytochrome c ou du Nitro blue tetrazolium chloride, par les $O_2^{\bullet -}$, en suivant l'augmentation d'absorbance à 550 nm (coefficient d'extinction molaire du cytochrome c : $\varepsilon_{550}$= 21,1 $mM^{-1}.cm^{-1}$).

Ce test est effectué sur 10μg de protéines membranaires de BL21 (DE3) pLyS surexprimant SpNox dans un volume total de 1mL. Le spectrophotomètre est utilisé pour une cinétique de 5min et à une longueur d'onde de 550nm.

Le tampon de la réaction est le tampon de resuspension des membranes (50mM Tris pH7)

Le blanc est effectué sur 10μg de membrane de BL21 (DE3) pLyS surexprimant SpNox et 100μM cytochrome c. La réaction est ensuite déclenchée par l'ajout de 100μM FAD et 200μM NADPH.

L'activité NADPH oxydase est ensuite quantifiée par le calcul de la pente de la courbe ainsi obtenue (Figure 4).

A partir de 10 μg de protéines membranaires de BL21 (DE3) pLyS surexprimant SpNox, le rapport d(DO)/D(t) obtenu est entre 0,1 et 0,5.

Une mesure de l'activité NADPH oxydase des protéines membranaires de BL21 (DE3) pLysS ne surexprimant pas SpNox (transformées par le vecteur vide) sur la réduction du cytochrome c permet de confirmer que la réduction observée au préalable est bien due à la présence de SpNox dans les membranes

**Exemple 4 : Protocole de mesure de l'activité NADPH oxydase de SpNox par colorimétrie**

Ce test est effectué sur 1μg SpNox dans un volume total de 1mL en utilisant le spectrophotomètre pour une cinétique de 5min et à une longueur d'onde de 550nm.

Le tampon de la réaction est le tampon de conservation de la protéine (50mM Tris pH7, 300mM NaCl, 0,03 ± 0,02 mM MNG3)

Le blanc est effectué sur 1ug SpNox et 100μM cytochrome c. La réaction est ensuite déclenchée par l'ajout de 100μM FAD et 200μM NADPH.

L'activité NADPH oxydase est ensuite mesurée par l'intermédiaire de la pente de la courbe ainsi obtenue. Le résultat obtenu est de 24 nanomoles de cytochrome c réduit par minute par microgramme de SpNox (ou de manière plus générale, entre 12 et 60 nanomoles de cytochrome c réduit par minute par microgramme de *Sp*Nox).

Cette activité peut être mesurée à différentes concentrations en NaCl (0 - 1M), en KCl et en MNG3.

**Exemple 5 : Protocole de mesure de l'activité NADPH oxydase de membranes de PLB985 enrichies en Nox eucaryote par colorimétrie (au spectrophotomètre)**

Le protocole suivant, décrit pour les cellules PLB985, peut être appliqué à d'autres lignées cellulaires comme les HEK 293 (Human Embryonic Kidney) et les PMN (Polymorphonuclear leukocytes).

Ce test est effectué sur 30 à 100ug de membranes de PLB985 dans un volume total de 1000μL.

Le tampon de la réaction est le PBS 1X.

Évaluation de l'effet d'une molécule A sur l'activité NADPH oxydase de Nox eucaryote

30-100μg de membrane de PLB985 sont incubées avec des concentrations croissantes d'une molécule A pour 5 - 20min à 25°C dans un volume de 100ul. 0,5-4mM d'acide arachidonique, 10-30μM de FAD et 100-500nM trimère (trimère non prénylée, rac 1Q61L) sont rajoutés au mélange réactionnel et incubés avec les membranes pendant 5-20min à 25°C selon le protocole décrit dans Mizrahi et al., A prenylated p47phox-p67phox-Racl chimera is a Quintessential NADPH oxidase activator: membrane association and functional capacity. J Biol Chem, 2010. 285(33): p. 25485-99.

La réaction est ensuite déclenchée par l'ajout de 900μL de PBS contenant le substrat de la NADPH oxydase, 100-400 μM NADPH (final) et 100 μM final du cytochrome c (ou du NBT) qui va permettre le suivi de la réaction.

Après homogénéisation, la cinétique de la réaction à 550nm est suivie pendant 5min.

**Exemple 6 : protocole de mesure de l'activité NADPH oxydase de *Sp*Nox par chimiluminescence et en utilisant un lecteur de plaque**

Cette technique repose sur le principe de dissipation de l'énergie par émission de lumière par un composé lorsqu'il retourne d'un état excité vers un état fondamental. La réaction de chimiluminescence appliquée repose sur une réaction d'oxydation du luminol (5-Amino-2,3-dihydro-1,4-phthalazinedione) dépendante de la peroxydase HRPO (horseradish peroxidase). Le retour du luminol excité vers son état fondamental s'accompagne d'une émission de lumière quantifiée par un luminomètre. L'émission de lumière est directement proportionnelle à l'activité NADPH oxydase.

Ce test est effectué sur 0,1-1μg de SpNox dans un volume total de 100 - 200μL. Le lecteur de plaque est utilisé pour une cinétique de 5min-20min.

Le tampon de la réaction est 50mM Tris pH7, 300mM NaCl, 0,03 ± 0,02 mM MNG3.

Évaluation de l'effet d'une molécule A sur l'activité NADPH oxydase de SpNox

0,1-1μg SpNox sont incubés avec des concentrations croissantes d'une molécule A pendant 5-20min à 25°C. 10-100μM luminol et 10-50U/ml HRPO sont rajoutés au mélange réactionnel et incubés avec la protéine *Sp*Nox et la molécule A à 25°C à l'abri de la lumière et pendant 5min.

La réaction est ensuite déclenchée par l'ajout de 20-50 μM FAD et 20-150μM NADPH. Pour chaque molécule A testée, il convient de faire 2 contrôles :

1. En remplaçant la protéine SpNox avec 50-500μM pyrogallol (sans l'ajout du FAD ni du NADPH) qui va s'auto-oxyder en présence d'oxygène en libérant des ions superoxydes en solution.
2. En absence de *Sp*Nox dans le mélange réactionnel et ceci afin de vérifier que la chemiluminescence mesurée est bien attribuée à SpNox et non pas à la molécule A.

L'incubation de SpNox avec la molécule A et avec le luminol et la HRPO peut se faire simultanément.

**Exemple 7 : Protocole de mesure de l'activité NADPH oxydase de SpNox par colorimétrie et en utilisant un lecteur de plaque**

L'activité oxydase est déterminée par mesure de la cinétique de réduction du cytochrome c (ou du Nitro blue tetrazolium chloride) par les $O_2^{\bullet-}$, en suivant l'augmentation d'absorbance à 550 nm (coefficient d'extinction molaire du cytochrome c: $\varepsilon_{550}$ = 21,1 $mM^{-1}.cm^{-1}$).

Ce test est effectué sur 0,1 - 1μg SpNox dans un volume total de 100 - 200μL. Le lecteur de plaque est utilisé pour une cinétique de 5 - 20min et à une longueur d'onde de 550nm.

Le tampon de la réaction est le tampon de conservation de SpNox (50mM Tris pH7, 300mM NaCl, 0,03mM ± 0,02 mM MNG3)

Évaluation de l'effet d'une molécule A sur l'activité NADPH oxydase de SpNox

0,1-1μg SpNox sont incubés avec des concentrations croissantes d'une molécule A pour 5-20min à 25°C. 10 - 30μM cytochrome c sont rajoutés au mélange réactionnel et la réaction est ensuite déclenchée par l'ajout de 20-50 μM FAD et 20-150 μM NADPH.

L'activité NADPH oxydase est ensuite quantifiée par le calcul de la pente de la courbe obtenue suite à la réduction du cytochrome c (ou du NBT).

Pour chaque molécule A testée, il convient de faire 2 contrôles :

1. En remplaçant la protéine SpNox avec 50-500μM pyrogallol (sans l'ajout du FAD ni du NADPH) qui va s'auto-oxyder en présence d'oxygène en libérant des ions superoxydes en solution. Ce test va permettre de vérifier que l'effet de la molécule A est exercée sur SpNox et non pas sur les radicaux superoxydes qu'elle produit (effet scavenger).
2. En absence de SpNox dans le mélange réactionnel et ceci afin de vérifier que la chemiluminescence mesurée est bien attribuée à SpNox et non pas à la molécule A.

Cette activité peut être mesurée à différentes concentrations en NaCl (0-1M) et en MNG3.

Protocole général de mesure de la cinétique de réduction du NBT

L'activité oxydase est déterminée par mesure de la cinétique de réduction du NBT par les ions $O_2^{\bullet-}$, en suivant l'augmentation d'absorbance à 560 nm (coefficient d'extinction molaire du NBT : $\varepsilon_{560}$= 30 000 $mM^{-1}.cm^{-1}$). Il est également possible d'utiliser une longueur d'onde de 550 nm.

*Sp*Nox est incubée avec un mélange réactionnel contenant la molécule à tester, le NBT, le FAD et le tampon de conservation de la protéine pendant 5 à 20 min à 25 °C. La réaction est ensuite déclenchée par l'ajout du NADPH.

L'activité NADPH oxydase est ensuite quantifiée en point final par la détermination de l'intensité de la coloration comparée à un contrôle positif. Le contrôle positif est réalisé avec la protéine *Sp*Nox seule, sans molécule à tester.

Les composés présents dans chaque puits de la plaque sont, en concentration finale :

- 0,1 μg de SpNox ;
- 2 μM de FAD ;
- 125 μM de NBT ;
- 150 μM NADPH;
- Molécule A ;
- Tampon de conservation de *Sp*Nox qsp 200 μL.

Toutes les expériences ont été effectuées en triplicata et chaque molécule a été testée à 5 concentrations différentes. La gamme de concentration est volontairement large afin de pouvoir encadrer les éventuelles IC50 de chaque molécule. Les concentrations sélectionnées sont : 0,01 - 0,1 - 1 - 10 - 100 μM.

<u>Validation expérimentale du test de criblage à haut débit :</u>

Deux pré-tests sont effectués en présence de :

1- une molécule bio-active (effet inhibiteur) sur *Sp*Nox, le diphenyleneiodonium chloride (DPI) ;
2- une molécule bio-inactive (sans effet inhibiteur) sur *Sp*Nox, le DMSO.

La pertinence, la qualité et la faisabilité de l'approche haut débit sont évaluées par l'établissement d'un « Z' score » (Zhang et al., J Biomol Screen, 4(2) : 67-73, 1999) à savoir :

$$Z' = 1 - \frac{(3\sigma_{c+} - 3\sigma_{c-})}{|\mu_{c+} - \mu_{c-}|} ;$$

dans lequel :

$\sigma_{c+}/\sigma_{c-}$ : écart types des résultats obtenus pour une contrôle positif / négatif ;
$\mu_{c+}/\mu_{c-}$ : moyennes des résultats obtenus pour une contrôle positif / négatif.

Si Z' = 1, la pertinence, la qualité et la faisabilité du test de criblage sont idéales.
Si 0,5 < Z' < 1, la pertinence, la qualité et la faisabilité du test de criblage sont excellentes.
Si 0 < Z' < 0,5, la pertinence, la qualité et la faisabilité du test de criblage sont marginales.
Si Z' < 0, la pertinence, la qualité et la faisabilité du test de criblage ne peuvent être interprétées.
Chaque pré-test doit être effectué un minimum de 20 fois afin de dégager des valeurs significatives pour permettre le calcul du « Z' score ».
Dans le cas présent, le « Z' score » est compris entre 0,7 et 0,8 selon les expériences.
En conséquence, un test en plaque 96 puits avec *Sp*Nox avec une révélation par colorimétrie (NBT) constitue un test assez robuste et fiable pour être appliqué en haut débit.

<u>Criblage contre des molécules commerciales ciblant les NOX eucaryotes :</u>

Un total de 8 molécules a été choisi et testé, 7 inhibiteurs et 1 activateur de l'activité NADPH oxydase des NOX eucaryotes (Tableau 1).

**Tableau 1.**

| Nom | Formule | Propriété |
|---|---|---|
| Ebsélène | 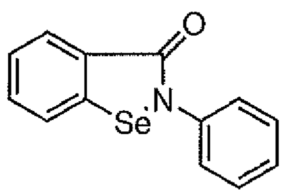 | Inhibiteur |

(suite)

| Nom | Formule | Propriété |
|---|---|---|
| Célastrol | 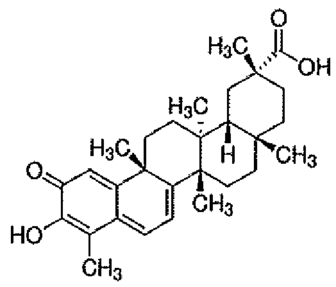 | Inhibiteur |
| Thr101 | 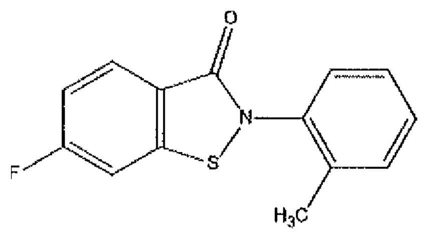 | Inhibiteur |
| VAS2870 | 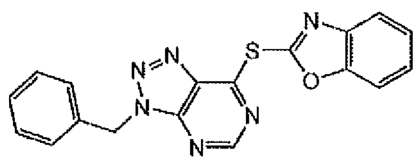 | Inhibiteur |
| Catéchine | 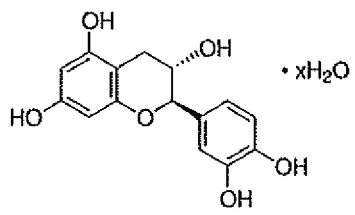 | Inhibiteur |
| Epicatéchine gallate | 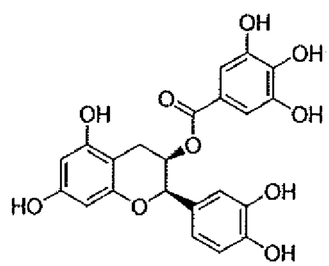 | Inhibiteur |
| DPI | 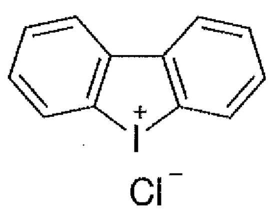 | Inhibiteur |
| Acide arachidonique (AA) | 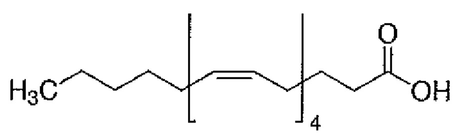 | Activateur |

Le suivi de l'activité NADPH oxydase de l'enzyme peut être réalisé en colorimétrie (ou en chimioluminescence)

(Figure 10).

Les résultats expérimentaux ont ainsi démontré que l'ebsélène, le célastrol, la Thr101, le VAS2870, la catéchine, l'épicatéchine gallate et le DPI sont des inhibiteurs de *Sp*Nox (Figures 11 à 17), et que l'acide arachidonique est un activateur de *Sp*Nox (Figure 18).

Ces résultats confirment donc qu'il est possible de cribler des molécules modulant l'activité des NOX eucaryotes en utilisant une protéine procaryote, telle que *Sp*Nox.

Par ailleurs, ces résultats indiquent également que les protéines NOX procaryotes peuvent également être utilisées pour identifier des molécules anti-bactériennes, que lesdites molécules aient ou non un effet sur les protéines NOX eucaryotes.

En effet, les molécules ayant un effet activateur sur les protéines NOX procaryotes sont susceptibles d'avoir un effet bactéricide ou bactériostatique sur les bactéries, en augmentant la production de ROS dans les bactéries.

**Exemple 8 : Protocole de mesure de l'activité NADPH oxydase de cellules PLB985 surexprimant une Nox eucaryote, par chimiluminescence et en utilisant un lecteur de plaque**

Cette technique repose sur le principe de dissipation de l'énergie par émission de lumière par un composé lorsque qu'il retourne d'un état excité vers son état initial. La réaction de chimiluminescence appliquée repose sur une réaction d'oxydation du luminol (5-Amino-2,3-dihydro-1,4-phthalazinedione) dépendante de la peroxydase HRPO (horseradish peroxidase) et de la peroxydase intrinsèque MPO. Le retour du luminol excité vers son état fondamental s'accompagne d'une émission de lumière quantifiée par un luminomètre. L'émission de lumière est directement proportionnelle à l'activité NADPH oxydase selon le protocole décrit dans Dahlgren, C. and A. Karlsson, Respiratory burst in human neutrophils. J Immunol Methods, 1999. 232(1-2): p. 3-14.

Ce test est effectué sur 5 x $10^5$ cellules dans un volume total de 250μL. Le lecteur de plaque est utilisé pour une cinétique de 15-45min.

Le tampon de la réaction est le PBS.

Évaluation de l'effet d'une molécule A sur l'activité NADPH oxydase de Nox eucaryote

5 x $10^5$ cellules PLB985 sont incubées avec des concentrations croissantes d'une molécule A pour 5-20 min à 25°C. 20mM glucose, 20μM luminol et 10 U/ml HRPO sont rajoutés au mélange réactionnel et incubés avec les cellules et la molécule A à 25°C à l'abri de la lumière et pendant 5min.

La réaction est ensuite déclenchée par l'ajout d'un activateur soluble de la NADPH oxydase :

- PMA 80 ng / ml
- FMLP 400 nM
- zymosan 0.6 mg / ml

Après homogénéisation, la cinétique de la réaction est suivie pendant 15-45 min.

Choisir le programme en fonction de l'activateur utilisé dans l'expérience :

- Programme zymosan : lecture pendant 30 min avec une mesure toutes les 15 secondes après stimulation par le ZOP (OPsonysed Zymosan)
- Programme PMA : lecture pendant 30 min avec une mesure toutes les 15 secondes après stimulation par le PMA
- Programme fMLP : lecture pendant 15 min avec une mesure toutes les 5 secondes après stimulation par le fMLP.

On obtient des vitesses de 0,00015 à 0,0005 mM de cytochrome c réduit par minutes pour 5 microgrammes de protéines membranaires totales.

**Exemple 9 : Protocole de mesure de l'activité NADPH oxydase de membrane de PLB985 enrichies en Nox eucaryote, par colorimétrie et en utilisant un lecteur de plaque**

Ce test est effectué sur 4-20ug de membrane de PLB985 dans un volume total de 200μL. Le lecteur de plaque est utilisé pour une cinétique de 5min.

Le tampon de la réaction est le PBS 1X.

Évaluation de l'effet d'une molécule A sur l'activité NADPH oxydase de Nox eucaryotique

4-20 ug de membrane de PLB985 sont incubées avec des concentrations croissantes d'une molécule A pour

5 à 20min à 25°C dans un volume de 100ul. 0,10-1mM d'acide arachidonique, 10-50 μM de FAD et la trimère sont rajoutés au mélange réactionnel et incubés avec les membranes pendant 5-20min à 25°C selon le protocole décrit dans Mizrahi, A., et al., A prenylated p47phox-p67phox-Rac1 chimera is a Quintessential NADPH oxidase activator: membrane association and functional capacity. J Biol Chem, 2010. 285(33): p. 25485-99.

La réaction est ensuite déclenchée par l'ajout de 100 μL de PBS contenant le substrat de la NADPH oxydase, 100-400 μM NADPH et 100 μM du cytochrome c (ou NBT) qui va permettre le suivi de la réaction.

Après homogénéisation, la cinétique de la réaction à 550nm est suivie pendant 5min.

## Revendications

1. Utilisation *in vitro, ex vivo* ou *in vivo,* comme outil de recherche pour le criblage de molécules présentant un intérêt thérapeutique, d'une protéine Nox ayant :

   ◦ un domaine comprenant de 3 à 7 hélices transmembranaires,

   et possédant une séquence en acides aminés comprenant :

   ◦ au moins 2 motifs bis-histidyl, chacun des motifs bis-histidyl étant constitué de 2 résidus histidine séparés par 12, 13 ou 14 résidus d'acides aminés, lesdits 2 motifs bis-histidyl étant situés dans la partie membranaire et séparés par une hélice transmembranaire, et
   ◦ au moins un premier motif constitué de la séquence :
   [G/S/A]-[Q/D]-F-[AN/T/L/F]-[F/Y/W/UR]-[LN/I/F/W] (SEQ ID NO: 1), et
   ◦ au moins un deuxième motif constitué de la séquence :
   [P/A/S/E/F]-H-[P/S/A]-F-[T/S]-[L/I/MN] (SEQ ID NO: 2), et
   ◦ au moins un troisième motif constitué de la séquence :
   [K/R]-X-X-G-[D/G]-X-[T/S] (SEQ ID NO: 3), X représentant n'importe quel acide aminé naturel, et
   ◦ au moins un quatrième motif constitué de la séquence :
   [G-[I/SN/L]-G-[V/I/A/F]-[T/A/S]-[P/Y/T/A] (SEQ ID NO: 4),

   ladite protéine Nox n'étant pas utilisée *in vivo* chez l'homme.

2. Utilisation selon la revendication 1, dans laquelle, dans la séquence d'acides aminés de ladite protéine Nox :

   ledit premier motif est choisi parmi le groupe constitué des séquences : SEQ ID NO : 8, SEQ ID NO : 9 et SEQ ID NO : 10, et/ou
   ledit deuxième motif est choisi parmi le groupe constitué des séquences : SEQ ID NO: 11, SEQ ID NO: 12, SEQ ID NO: 13 et SEQ ID NO: 14, et/ou
   ledit troisième motif est choisi parmi le groupe constitué des séquences : SEQ ID NO : 15, SEQ ID NO: 16, SEQ ID NO: 17, SEQ ID NO: 18, SEQ ID NO: 19, SEQ ID NO : 20, et/ou
   ledit quatrième motif est choisi parmi le groupe constitué des séquences : SEQ ID NO: 21, SEQ ID NO: 22 et SEQ ID NO: 23.

3. Utilisation selon l'une quelconque des revendications 1 ou 2, dans laquelle la séquence en acides aminés de ladite protéine Nox comprend, ou consiste en, une séquence ayant au moins 90 % d'identité, de préférence au moins 95% d'identité, avec l'une des séquences choisies dans le groupe constitué de : SEQ ID NO: 24, SEQ ID NO: 25, SEQ ID NO: 26, SEQ ID NO: 27, SEQ ID NO: 28 et SEQ ID NO: 29.

4. Utilisation selon l'une quelconque des revendications 1 ou 2, dans laquelle la séquence en acides aminés de ladite protéine Nox comprend, ou consiste en, une séquence choisie parmi le groupe constitué des séquences : SEQ ID NO : 24 à 351.

5. Utilisation selon l'une quelconque des revendications 1 à 4, dans laquelle ladite protéine Nox possède une activité NADPH oxydase entraînant une production de ROS en présence d'un donneur d'électrons,
   ladite activité NADPH oxydase étant susceptible d'être détectée dans un échantillon comprenant ladite protéine par l'intermédiaire d'une réaction montrant la production d'ion superoxyde.

6. Utilisation selon l'une quelconque des revendications 1 à 5, dans laquelle ladite protéine Nox constitue une cible

thérapeutique modèle.

7. Utilisation selon l'une quelconque des revendications 1 à 6, dans laquelle ladite protéine Nox est une protéine procaryote, ladite protéine Nox procaryote étant utilisée pour identifier des molécules capables de moduler l'activité NADPH oxydase d'une protéine Nox eucaryote.

8. Utilisation selon l'une quelconque des revendications 1 à 7, dans laquelle ladite protéine Nox est adsorbée sur un support.

9. Utilisation selon l'une quelconque des revendications 1 à 5, pour le criblage de molécules susceptibles de présenter des propriétés antibiotiques.

10. Utilisation selon la revendication 5, pour le criblage de molécules susceptibles de présenter des propriétés antibiotiques capables de stimuler la production de ROS par ladite protéine Nox.

11. Utilisation *in vitro, ex vivo* ou *in vivo*, comme outil de recherche pour le criblage de molécules présentant un intérêt thérapeutique :

- d'un acide nucléique codant une protéine Nox telle que définie dans l'une des revendications 1 à 5,
- d'un vecteur d'expression contenant ledit acide nucléique, ou
- d'une cellule contenant ledit vecteur d'expression,

ledit acide nucléique, ledit vecteur d'expression et la dite cellule n'étant pas utilisés *in vivo* chez l'homme.

12. Utilisation selon la revendication 11, dans laquelle ledit acide nucléique comprend ou consiste en une séquence choisie parmi le groupe constitué des séquences SEQ ID NO : 352 à 363.

13. Utilisation selon la revendication 11, dans laquelle ledit vecteur d'expression comprend ou consiste en une séquence choisie parmi le groupe constitué des séquences SEQ ID NO : 364 à 369.

14. Utilisation selon la revendication 11, dans laquelle ladite cellule est une bactérie.

15. Utilisation selon la revendication 14, selon laquelle ladite cellule est une bactérie choisie parmi le groupe constitué d'*Escherichia coli, Streptococcus pneumoniae, Pseudomonas aeruginosa, Vibrio cholerae, Salmonella enterica et Bordetella pertussis.*

## Patentansprüche

1. Verwendung *in vitro*, *ex vivo* oder *in vivo,* als Suchwerkzeug zur Siebung von ein therapeutisches Interesse aufweisenden Molekülen, eines Nox-Proteins mit:

- einem Bereich, 3 bis 7 transmembranäre Helices umfassend,

und verfügend über eine Aminosäuresequenz, Folgendes umfassend:

- wenigstens 2 Bis-histidyl-Motive, wobei jedes der Bis-histidyl-Motive aus 2 Histidinresten besteht, getrennt nach 12, 13 oder 14 Aminosäureresten, wobei sich die 2 Bis-histidyl-Motive in dem Membranteil befinden und durch eine transmembranäre Helix getrennt sind, und
- wenigstens ein erstes Motiv, bestehend aus der Sequenz:
[G/S/A]-[Q/D]-F-[AN/T/L/F]-[F/Y/W/L/R]-[LN/I/F/W] (SEQ ID NO: 1), und
- wenigstens ein zweites Motiv, bestehend aus der Sequenz:
[P/A/S/E/F]-H-[P/S/A]-F-[T/S]-[L/I/MN] (SEQ ID NO: 2), und
- wenigstens ein drittes Motiv, bestehend aus der Sequenz:
[K/R]-X-X-G-[D/G]-X-[T/S] (SEQ ID NO: 3), wobei X für eine beliebige natürliche Aminosäure steht, und
- wenigstens ein viertes Motiv, bestehend aus der Sequenz:
[G-[I/SN/L]-G-[V/I/A/F]-[T/A/S]-[P/Y/T/A] (SEQ ID NO: 4),

wobei das Nox-Protein nicht *in vivo* bei einem Menschen verwendet wird.

**2.** Verwendung nach Anspruch 1, wobei in der Aminosäuresequenz des Nox-Proteins:

das erste Motiv ausgewählt ist aus der Gruppe bestehend aus den Sequenzen: SEQ ID NO: 8, SEQ ID NO: 9 und SEQ ID NO: 10, und/oder
das zweite Motiv ausgewählt ist aus der Gruppe bestehend aus den Sequenzen: SEQ ID NO: 11, SEQ ID NO: 12, SEQ ID NO: 13 und SEQ ID NO: 14, und/oder
das dritte Motiv ausgewählt ist aus der Gruppe bestehend aus den Sequenzen: SEQ ID NO: 15, SEQ ID NO: 16, SEQ ID NO: 17, SEQ ID NO: 18, SEQ ID NO: 19, SEQ ID NO: 20, und/oder
das vierte Motiv ausgewählt ist aus der Gruppe bestehend aus den Sequenzen: SEQ ID NO: 21, SEQ ID NO: 22 und SEQ ID NO: 23.

**3.** Verwendung nach einem der Ansprüche 1 oder 2, wobei die Aminosäuresequenz des Nox-Proteins eine Sequenz mit wenigstens 90 % Identität, bevorzugt 95 % Identität, mit einer der Sequenzen ausgewählt aus der Gruppe bestehend aus: SEQ ID NO: 24, SEQ ID NO: 25, SEQ ID NO: 26, SEQ ID NO: 27, SEQ ID NO: 28 und SEQ ID NO: 29, umfasst oder aus dieser besteht.

**4.** Verwendung nach einem der Ansprüche 1 oder 2, wobei die Aminosäuresequenz des Nox-Proteins eine Sequenz ausgewählt aus der Gruppe bestehend aus den Sequenzen: SEQ ID NO: 24 bis 351 umfasst oder aus dieser besteht.

**5.** Verwendung nach einem der Ansprüche 1 bis 4, wobei das Nox-Protein über eine NADPH-Oxydasenaktivität verfügt, in Anwesenheit eines Elektronenspenders zu einer Produktion von ROS führend,
wobei die NADPH-Oxydase wahrscheinlich in einer das Protein enthaltenden Probe erkannt wird mittels einer Reaktion, die die Produktion von Superoxydionen zeigt.

**6.** Verwendung nach einem der Ansprüche 1 bis 5, wobei das Nox-Protein ein modellhaftes therapeutisches Ziel aufweist.

**7.** Verwendung nach einem der Ansprüche 1 bis 6, wobei das Nox-Protein ein Prokaryotprotein ist, das verwendet wird, um Moleküle zu identifizieren, fähig, die NADPH-Oxydasenaktivität mit einem Nox-Eukaryotprotein zu modifizieren.

**8.** Verwendung nach einem der Ansprüche 1 bis 7, wobei das Nox-Protein auf einem Träger absorbiert wird.

**9.** Verwendung nach einem der Ansprüche 1 bis 5 zur Siebung von Molekülen, die wahrscheinlich antibiotische Eigenschaften aufweisen.

**10.** Verwendung nach Anspruch 5, zur Siebung von Molekülen, die wahrscheinlich antibiotische Eigenschaften aufweisen, fähig, die ROS-Produktion durch das Nox-Protein zu stimulieren.

**11.** Verwendung, *in vitro, ex vivo* oder *in vivo* als Suchwerkzeug zur Siebung von ein therapeutisches Interesse aufweisenden Molekülen:

- einer Nukleinsäure, ein Nox-Protein codierend wie in einem der Ansprüche 1 bis 5 definiert,
- eines Expressionsvektors, die Nukleinsäure enthaltend, oder
- einer Zelle, den Expressionsvektor enthaltend,

wobei die Nukleinsäure, der Expressionsvektor und die Zelle nicht *in vivo* bei einem Menschen verwendet werden.

**12.** Verwendung nach Anspruch 11, wobei die Nukleinsäure eine Sequenz ausgewählt aus der Gruppe bestehend aus den Sequenzen SEQ ID NO: 352 bis 363 umfasst oder aus dieser besteht.

**13.** Verwendung nach Anspruch 11, wobei die Nukleinsäure eine Sequenz ausgewählt aus der Gruppe bestehend aus den Sequenzen SEQ ID NO: 364 bis 369 umfasst oder aus dieser besteht.

**14.** Verwendung nach Anspruch 11, wobei die Zelle eine Bakterie ist.

15. Verwendung nach Anspruch 14, wobei die Zelle eine Bakterie ist ausgewählt aus der Gruppe bestehend aus *Escherichia coli, Streptococcus pneumoniae, Pseudomonas aeruginosa, Vibrio cholerae, Salmonella enterica* und *Bordetella pertussis.*

**Claims**

1. Use *in vitro, ex vivo* or *in vivo,* as a research tool for screening molecules of therapeutic interest, of a Nox protein comprising:

   - a domain comprising from 3 to 7 transmembrane helices;

   and having an amino acids sequence comprising:

   - at least 2 bis-histidyl motifs, each of the bis-histidyl motifs consisting of 2 histidine residues separated by 12, 13 or 14 amino acid residues, said 2 bis-histidyl motifs being located in the membrane part and separated by a transmembrane helix, and
   - at least one first motif consisting of the sequence:
   [G/S/A]-[Q/D]-F-[A/V/T/L/F]-[F/Y/W/L/R]-[L/V/I/F/W] (SEQ ID NO: 1), and
   - at least one second motif consisting of the sequence:
   [P/A/S/E/F]-H-[P/S/A]-F-[T/S]-[L/I/M/V] (SEQ ID NO: 2) and
   - at least one third motif consisting of the sequence:
   [K/R]-X-X-G-[D/G]-X-[T/S] (SEQ ID NO: 3), wherein X represents any naturally occurring amino acid, and
   - at least one fourth motif consisting of the sequence:
   G-[I/S/V/L]-G-[V/I/A/F]-[T/A/S]-[P/Y/T/A] (SEQ ID NO: 4)

   wherein said Nox protein is not used *in vivo* in humans.

2. Use as claimed in claim 1, wherein, in the amino acid sequence of said Nox protein:

   said first motif is selected from the group consisting of the sequences: SEQ ID NO: 8, SEQ ID NO: 9 and SEQ ID NO: 10, and/or
   said second motif is selected from the group consisting of the sequences: SEQ ID NO: 11, SEQ ID NO: 12, SEQ ID NO: 13 and SEQ ID NO: 14, and/or
   said third motif is selected from the group consisting of the sequences: SEQ ID NO: 15, SEQ ID NO: 16, SEQ ID NO: 17, SEQ ID NO: 18, SEQ ID NO: 19, and SEQ ID NO: 20, and/or
   said fourth motif is selected from the group consisting of the sequences: SEQ ID NO: 21, SEQ ID NO: 22 and SEQ ID NO: 23.

3. Use as claimed in any one of claims 1 or 2, wherein the amino acid sequence of said Nox protein comprises, or consists of, a sequence having at least 90% identity, preferably at least 95% identity, with one of the sequences selected in the group consisting of: SEQ ID NO: 24, SEQ ID NO: 25, SEQ ID NO: 26, SEQ ID NO: 27, SEQ ID NO: 28 and SEQ ID NO: 29.

4. Use as claimed in any one of claims 1 or 2, wherein the amino acid sequence of said Nox protein comprises, or consists of, a sequence chosen from the group consisting of the sequences: SEQ ID NO: 24 to 351.

5. Use as claimed in any one of claims 1 to 4, wherein said Nox protein has a NADPH oxidase activity leading to production of ROS in the presence of an electron donor,
   said NADPH oxidase activity being detectable in a sample comprising said protein via a reaction showing production of superoxide ion.

6. Use as claimed in any one of claims 1 to 5, wherein said Nox protein constitutes a model therapeutic target.

7. Use as claimed in any one of claims 1 to 6, wherein said Nox protein is a prokaryotic protein, said prokaryotic Nox protein being used for identifying molecules capable of modulating the NADPH oxidase activity of a eukaryotic Nox protein.

8. Use as claimed in any one of claims 1 to 7, wherein said Nox protein is adsorbed on a support.

9. Use as claimed in any one of claims 1 to 5, for screening molecules likely to have antibiotic properties.

10. Use as claimed in claim 5, for screening molecules likely to have antibiotic properties capable of stimulating the production of ROS by said Nox protein.

11. Use *in vitro, ex vivo* or *in vivo,* as a research tool for screening molecules of therapeutic interest:

- of a nucleic acid coding for a Nox protein as defined in any of claims 1 to 5,
- of an expression vector containing said nucleic acid, or
- of a cell containing said expression vector,

said nucleic acid, said expression vector and said cell not being used *in vivo* in humans.

12. Use as claimed in claim 11, wherein said nucleic acid comprises or consists of a sequence selected from the group consisting of the sequences SEQ ID NO: 352 to 363.

13. Use as claimed in claim 11, wherein said expression vector comprises or consists of a sequence selected from the group consisting of the sequences SEQ ID NO: 364 to 369.

14. Use as claimed in claim 11, wherein said cell is a bacterium.

15. Use as claimed in claim 14, wherein said cell is a bacterium selected from the group consisting of *Escherichia coli, Streptococcus pneumoniae, Pseudomonas aeruginosa, Vibrio cholerae, Salmonella enterica* and *Bordetella pertussis.*

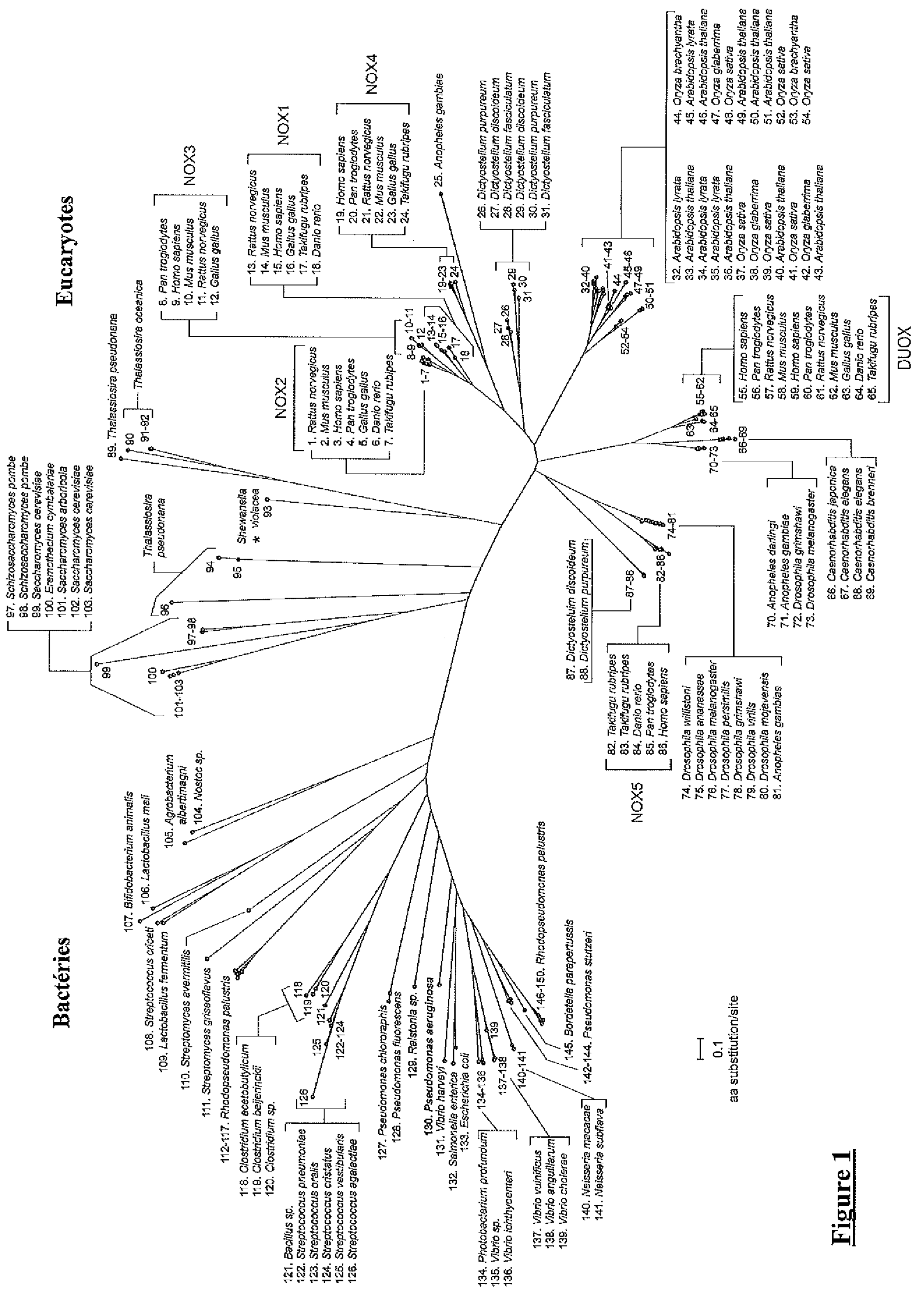
Eucaryotes
Bactéries
NOX1
NOX2
NOX3
NOX4
NOX5
DUOX
1. Rattus norvegicus
2. Mus musculus
3. Homo sapiens
4. Pan troglodytes
5. Gallus gallus
6. Danio rerio
7. Takifugu rubipes
8. Pan troglodytes
9. Homo sapiens
10. Mus musculus
11. Rattus norvegicus
12. Gallus gallus
13. Rattus norvegicus
14. Mus musculus
15. Homo sapiens
16. Gallus gallus
17. Takifugu rubripes
18. Danio rerio
19. Homo sapiens
20. Pan troglodytes
21. Rattus norvegicus
22. Mus musculus
23. Gallus gallus
24. Takifugu rubripes
25. Anopheles gambiae
26. Dictyostelium purpureum
27. Dictyostelium discoideum
28. Dictyostelium fasciculatum
29. Dictyostelium discoideum
30. Dictyostelium purpureum
31. Dictyostelium fasciculatum
32. Arabidopsis lyrata
33. Arabidopsis thaliana
34. Arabidopsis lyrata
35. Arabidopsis lyrata
36. Arabidopsis thaliana
37. Oryza sativa
38. Oryza glaberrima
39. Oryza sativa
40. Arabidopsis thaliana
41. Oryza sativa
42. Oryza glaberrima
43. Arabidopsis thaliana
44. Oryza brachyantha
45. Arabidopsis lyrata
46. Arabidopsis thaliana
47. Oryza glaberrima
48. Oryza sativa
49. Arabidopsis thaliana
50. Arabidopsis thaliana
51. Arabidopsis thaliana
52. Oryza sativa
53. Oryza brachyantha
54. Oryza sativa
55. Homo sapiens
56. Pan troglodytes
57. Rattus norvegicus
58. Mus musculus
59. Homo sapiens
60. Pan troglodytes
61. Rattus norvegicus
62. Mus musculus
63. Gallus gallus
64. Danio rerio
65. Takifugu rubripes
66. Caenorhabditis japonica
67. Caenorhabditis elegans
68. Caenorhabditis elegans
69. Caenorhabditis brenneri
70. Anopheles darlingi
71. Anopheles gambiae
72. Drosophila grimshawi
73. Drosophila melanogaster
74. Drosophila willistoni
75. Drosophila ananassae
76. Drosophila melanogaster
77. Drosophila persimilis
78. Drosophila grimshawi
79. Drosophila virilis
80. Drosophila mojavensis
81. Anopheles gambiae
82. Takifugu rubripes
83. Takifugu rubripes
84. Danio rerio
85. Pan troglodytes
86. Homo sapiens
87. Dictyostelium discoideum
88. Dictyostelium purpureum
89. Thalassiosira pseudonana
Thalassiosira oceanica
Thalassiosira pseudonana
Shewanella violacea
97. Schizosaccharomyces pombe
98. Schizosaccharomyces pombe
99. Saccharomyces cerevisiae
100. Eremothecium cymbalariae
101. Saccharomyces arboricola
102. Saccharomyces cerevisiae
103. Saccharomyces cerevisiae
104. Nostoc sp.
105. Agrobacterium albertimagni
106. Lactobacillus mali
107. Bifidobacterium animalis
108. Streptococcus criceti
109. Lactobacillus fermentum
110. Streptomyces avermitilis
111. Streptomyces griseoflavus
112-117. Rhodopseudomonas palustris
118. Clostridium acetobutylicum
119. Clostridium beijerinckii
120. Clostridium sp.
121. Bacillus sp.
122. Streptococcus pneumoniae
123. Streptococcus oralis
124. Streptococcus cristatus
125. Streptococcus vestibularis
126. Streptococcus agalactiae
127. Pseudomonas chlororaphis
128. Pseudomonas fluorescens
129. Ralstonia sp.
130. Pseudomonas aeruginosa
131. Vibrio harveyi
132. Salmonella enterica
133. Escherichia coli
134. Photobacterium profundum
135. Vibrio sp.
136. Vibrio ichthyoenteri
137. Vibrio vulnificus
138. Vibrio anguillarum
139. Vibrio cholerae
140. Neisseria macacae
141. Neisseria subflava
142-144. Pseudomonas stutzeri
145. Bordetella parapertussis
146-150. Rhodopseudomonas palustris
0.1
aa substitution/site
Figure 1

Protéine Nox humaine (*Nox2*)

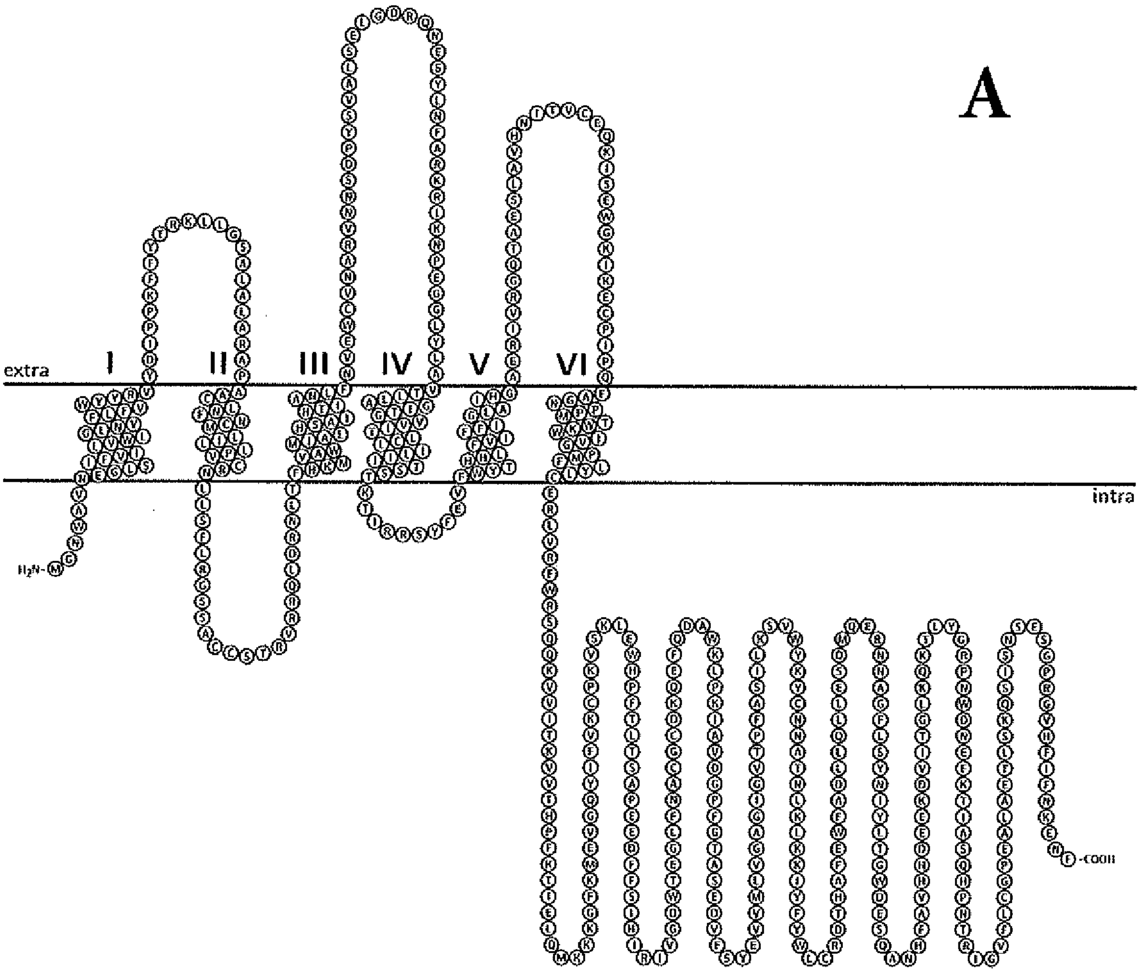


**Figure 2 (A)**

Protéine Nox de *Streptococcus pneumoniae* (*Sp*Nox)

# B

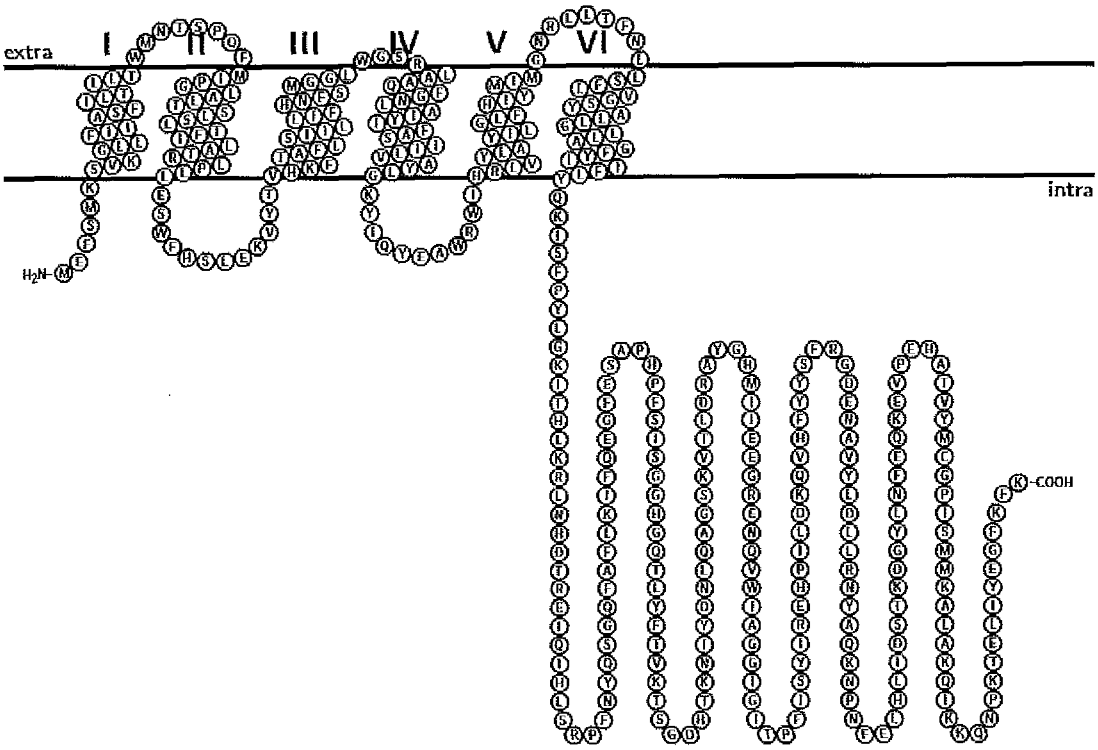


**Figure 2 (B)**

Protéine Nox de *Pseudomonas aeruginosa* (*Pa*Nox)

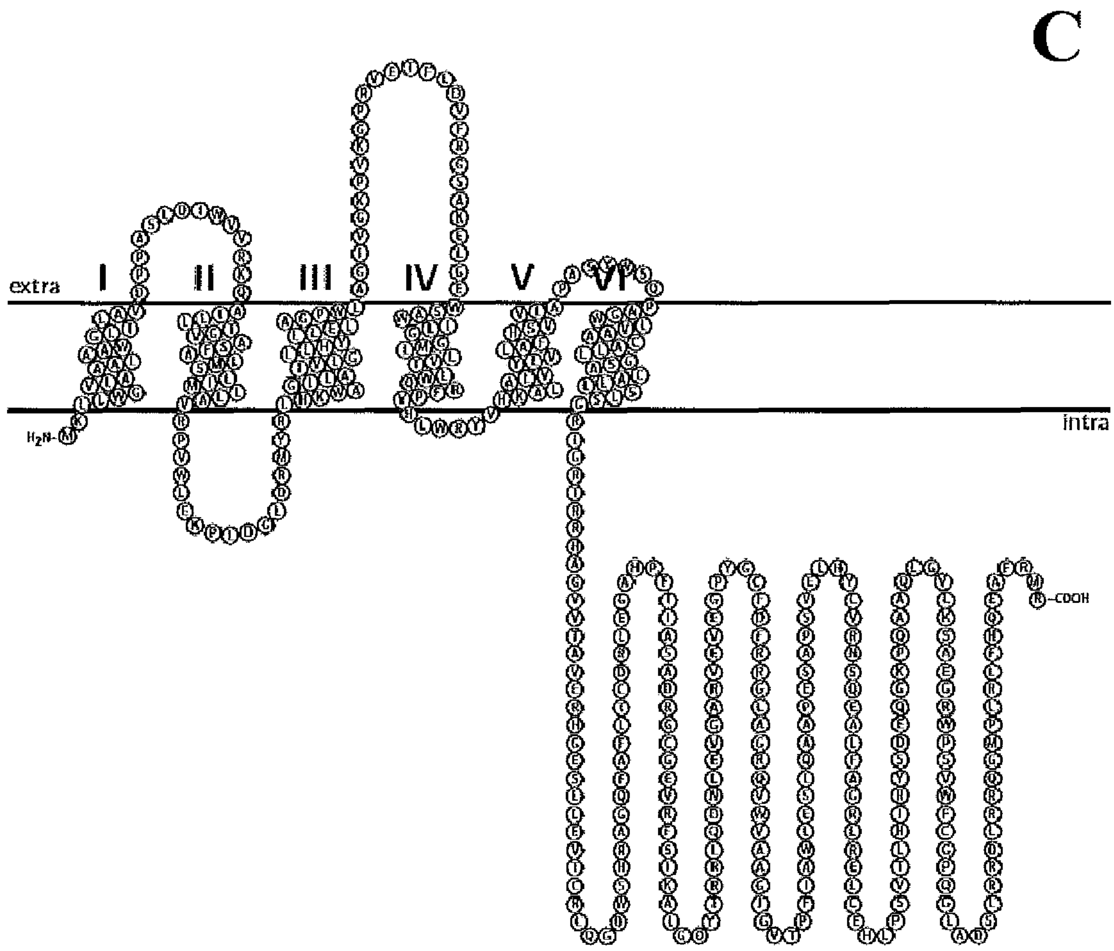


**Figure 2 (C)**

Protéine Nox de *Escherichia coli* (*Ec*Nox)

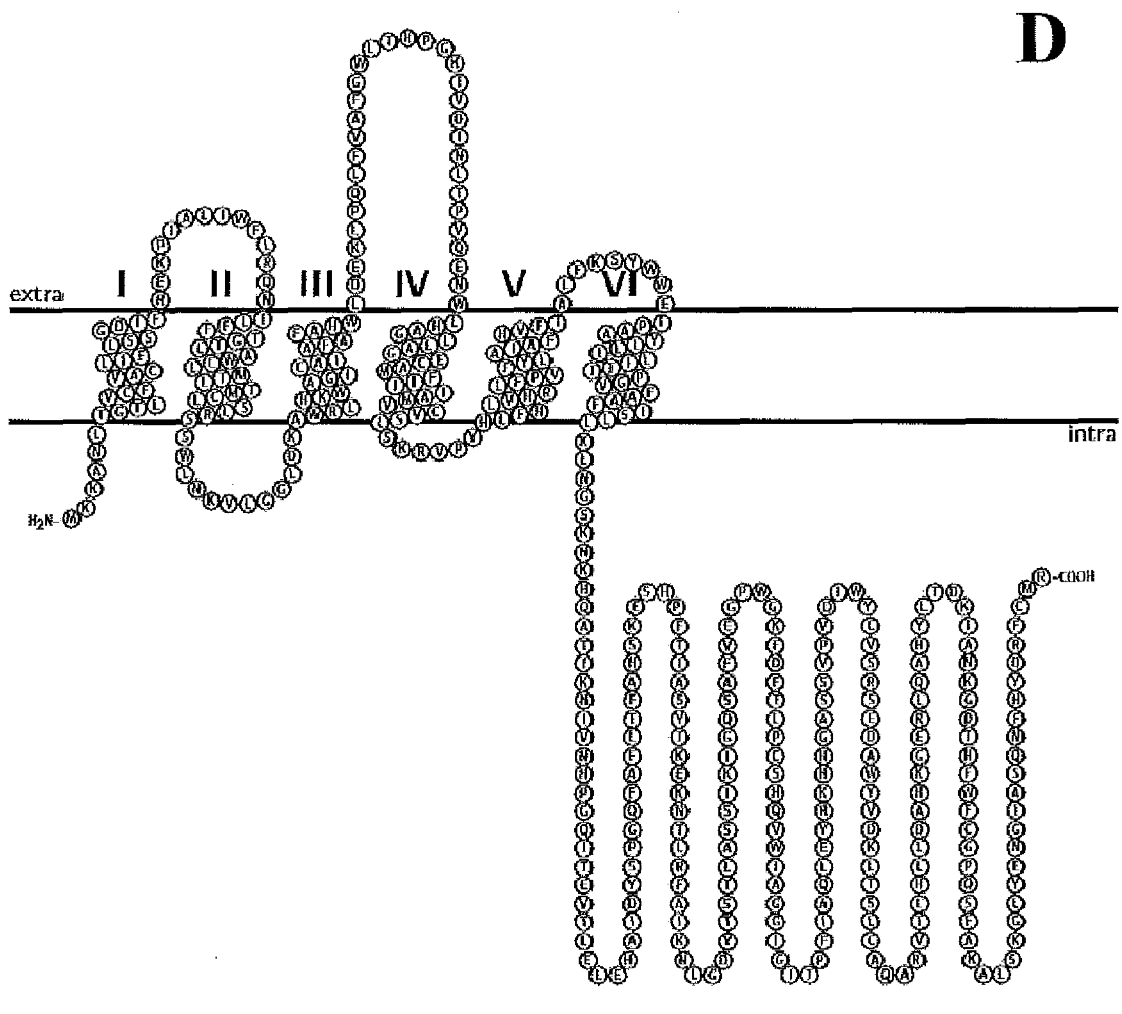


Figure 2 (D)

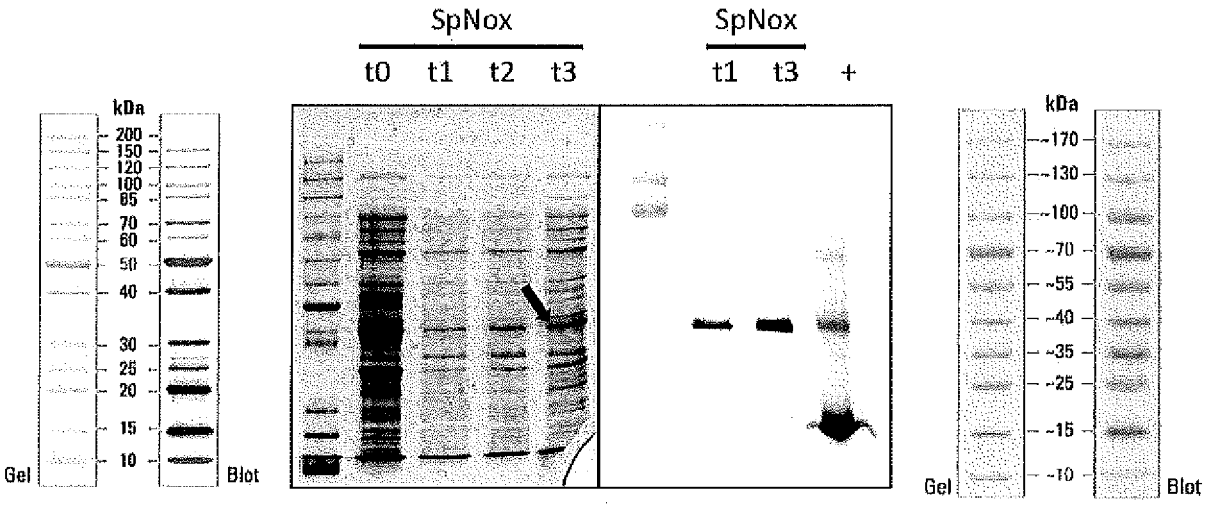
SpNox
t0
t1
t2
t3
SpNox
t1
t3
+
kDa
200
150
120
100
85
70
60
50
40
30
25
20
15
10
Gel
Blot
kDa
~170
~130
~100
~70
~55
~40
~35
~25
~15
~10
Gel
Blot

**Figure 3**

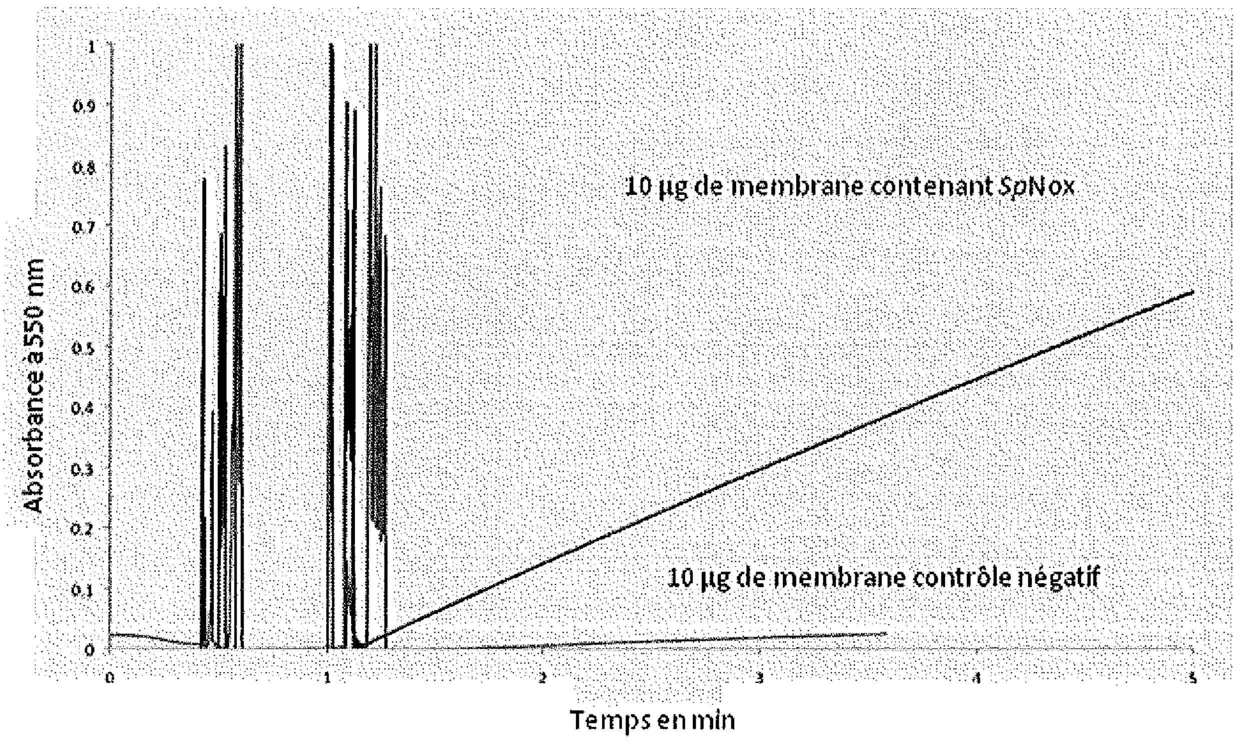
Absorbance à 550 nm
1
0.9
0.8
0.7
0.6
0.5
0.4
0.3
0.2
0.1
0
10 µg de membrane contenant *Sp*Nox
10 µg de membrane contrôle négatif
0
1
2
3
4
5
Temps en min

**Figure 4**

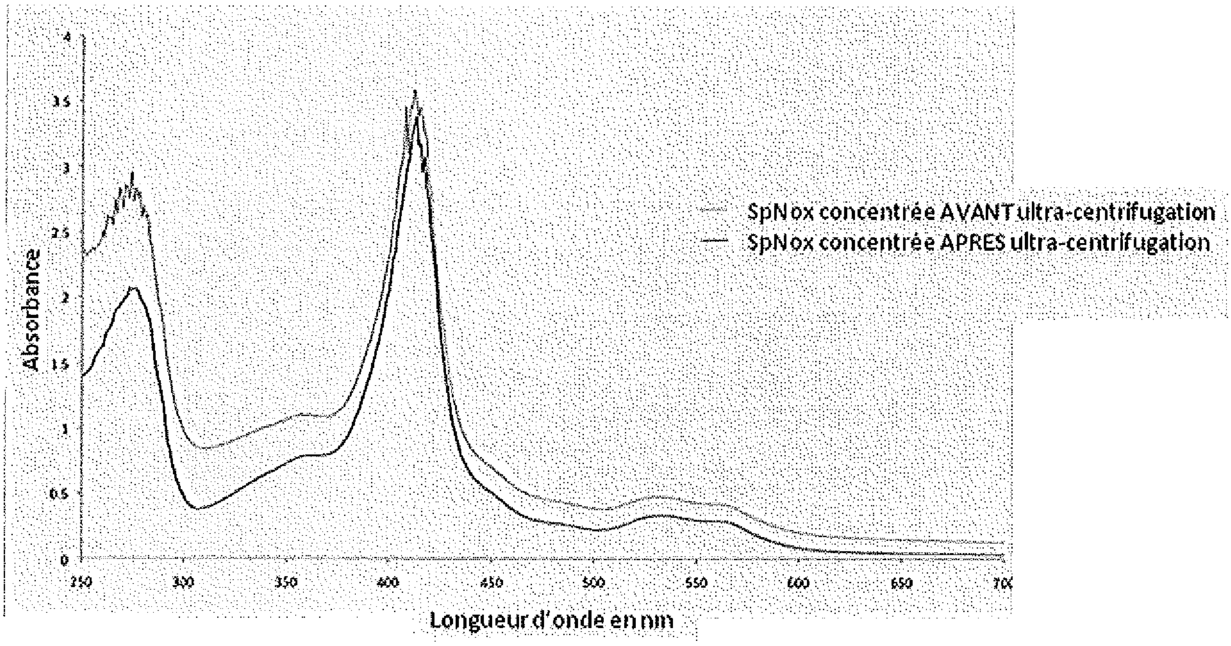
Absorbance
Longueur d'onde en nm
SpNox concentrée AVANT ultra-centrifugation
SpNox concentrée APRES ultra-centrifugation
0
0.5
1
1.5
2
2.5
3
3.5
4
250
300
350
400
450
500
550
600
650
700

**Figure 5**

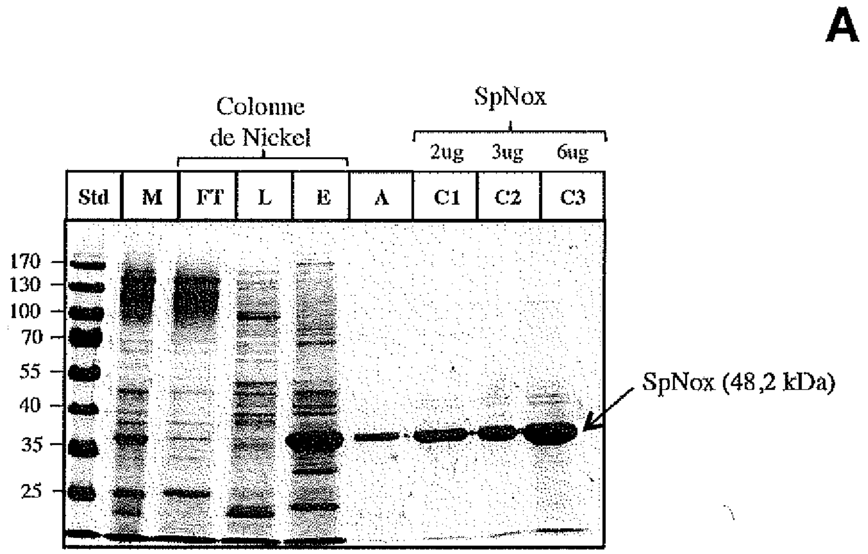
A
Colonne
de Nickel
SpNox
2ug
3ug
6ug
Std
M
FT
L
E
A
C1
C2
C3
170
130
100
70
55
40
35
25
SpNox (48,2 kDa)

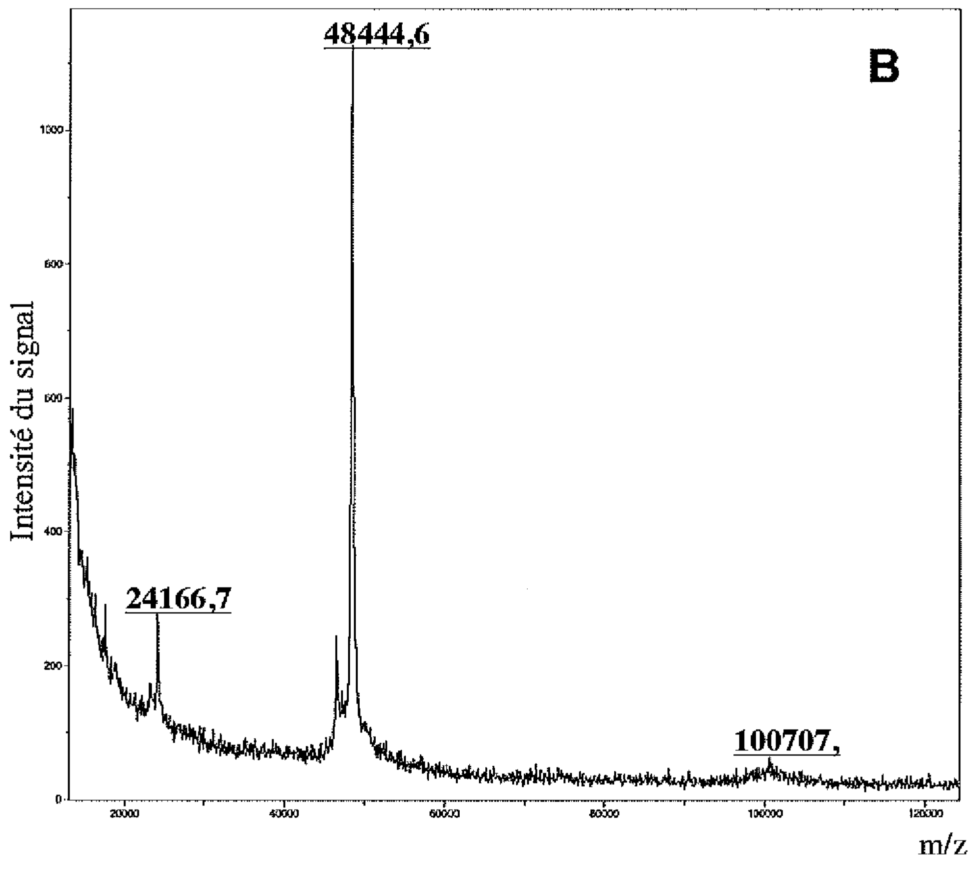
B
48444,6
24166,7
100707,
Intensité du signal
m/z

**Figure 6**

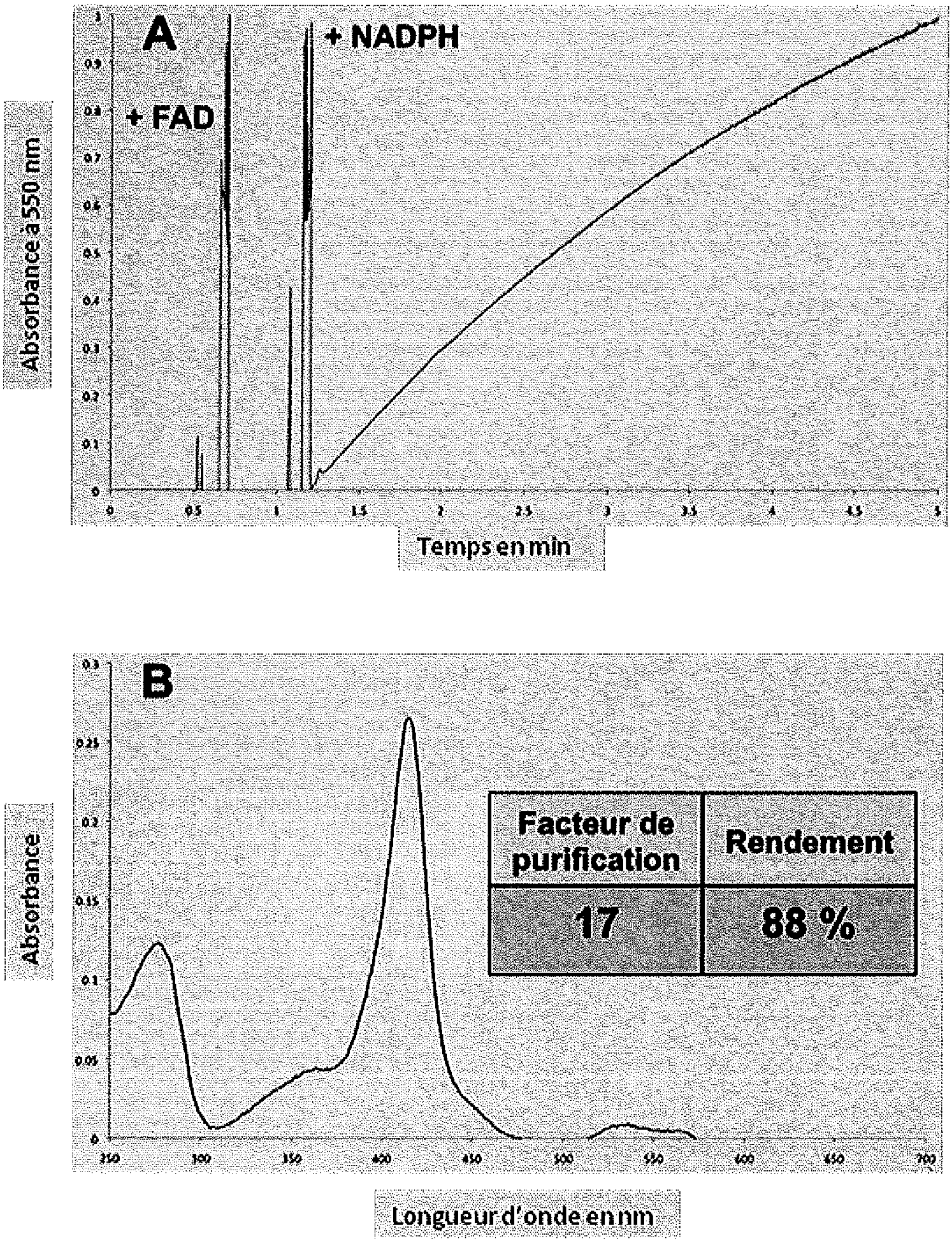
A
+ FAD
+ NADPH
Absorbance à 550 nm
Temps en min
B
Facteur de purification
Rendement
17
88 %
Absorbance
Longueur d'onde en nm

Figure 7

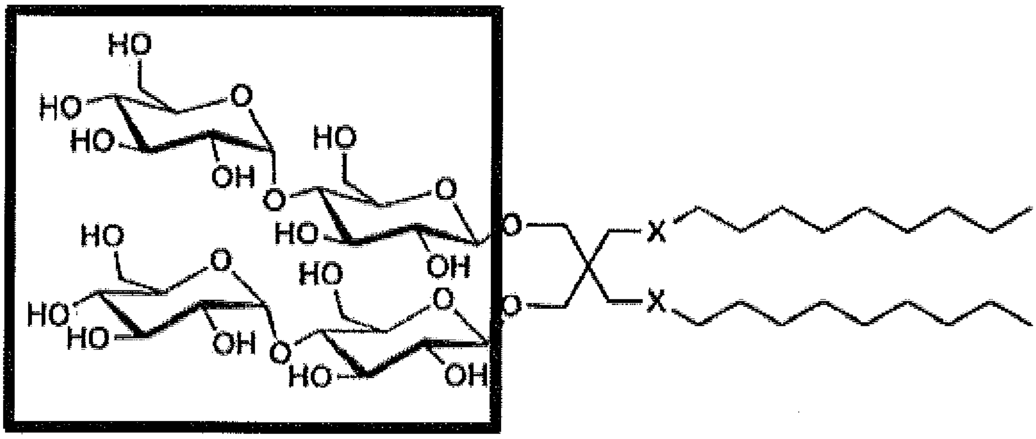

MNG-1: X = 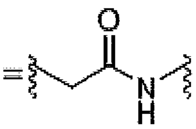 (CMC ~17 μM ; 0.0019 wt %)

MNG-2: X = 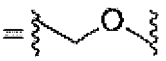 (CMC ~9 μM ; 0.0010 wt %)

MNG-3: X = Nothing (CMC ~10 μM ; 0.0010 wt %)

**Figure 8 (A)**

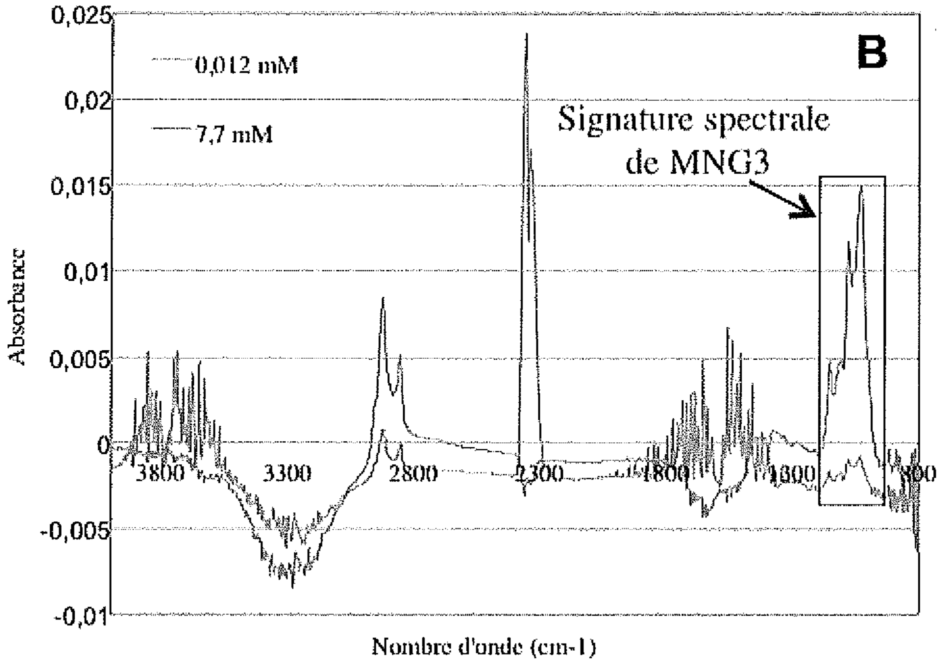
0,025
0,02
0,015
0,01
0,005
0
-0,005
-0,01
Absorbance
0,012 mM
7,7 mM
B
Signature spectrale
de MNG3
3800
3300
2800
2300
1800
1300
800
Nombre d'onde (cm-1)

**Figure 8**

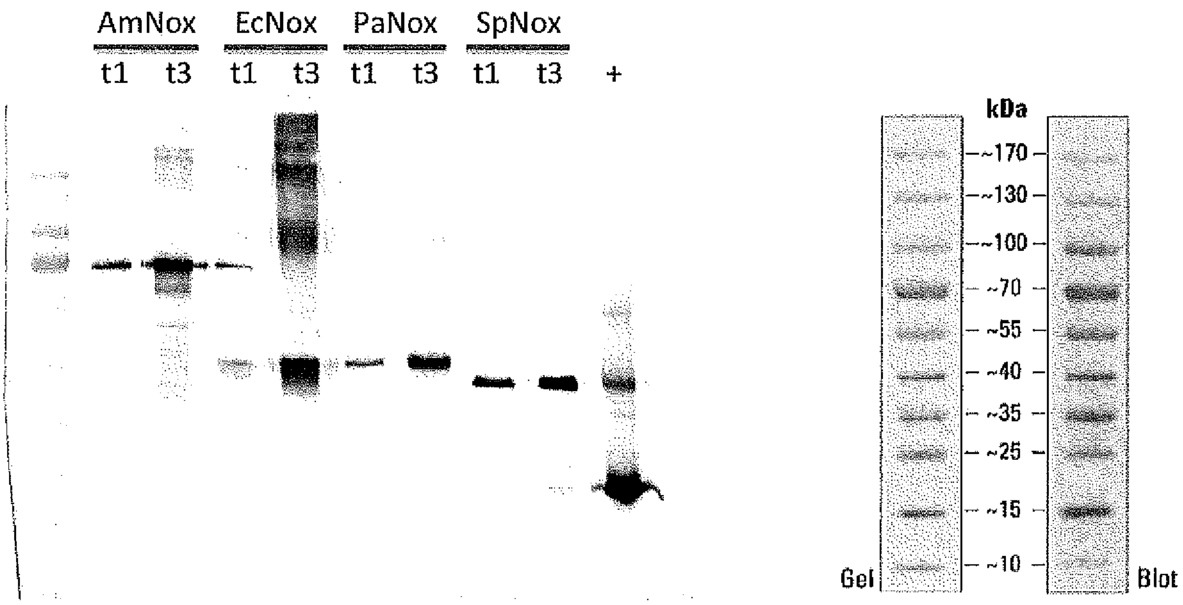


**Figure 9**

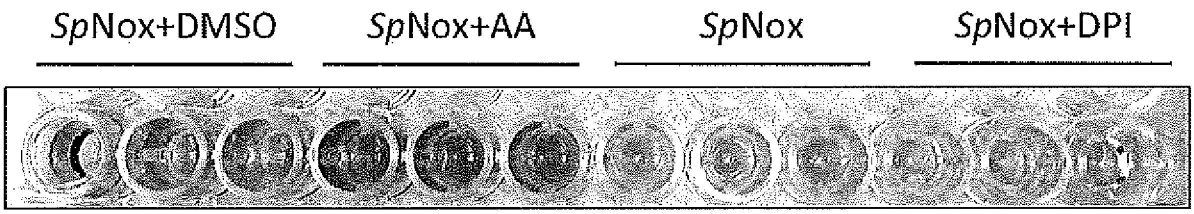


**Figure 10**

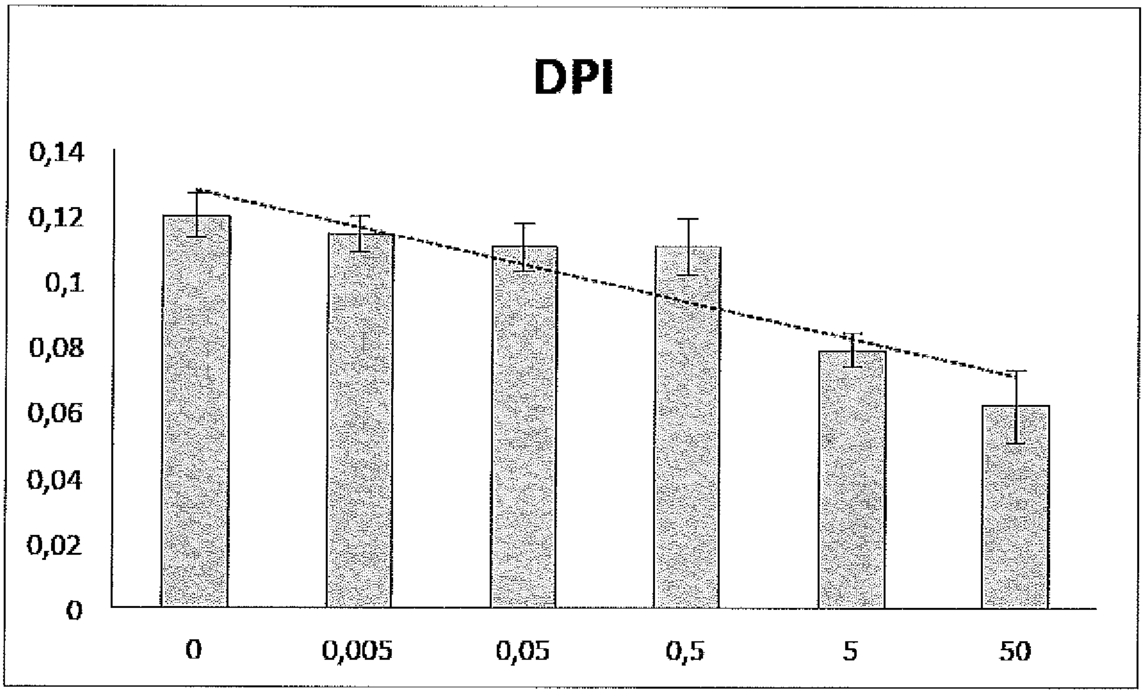


Figure 11

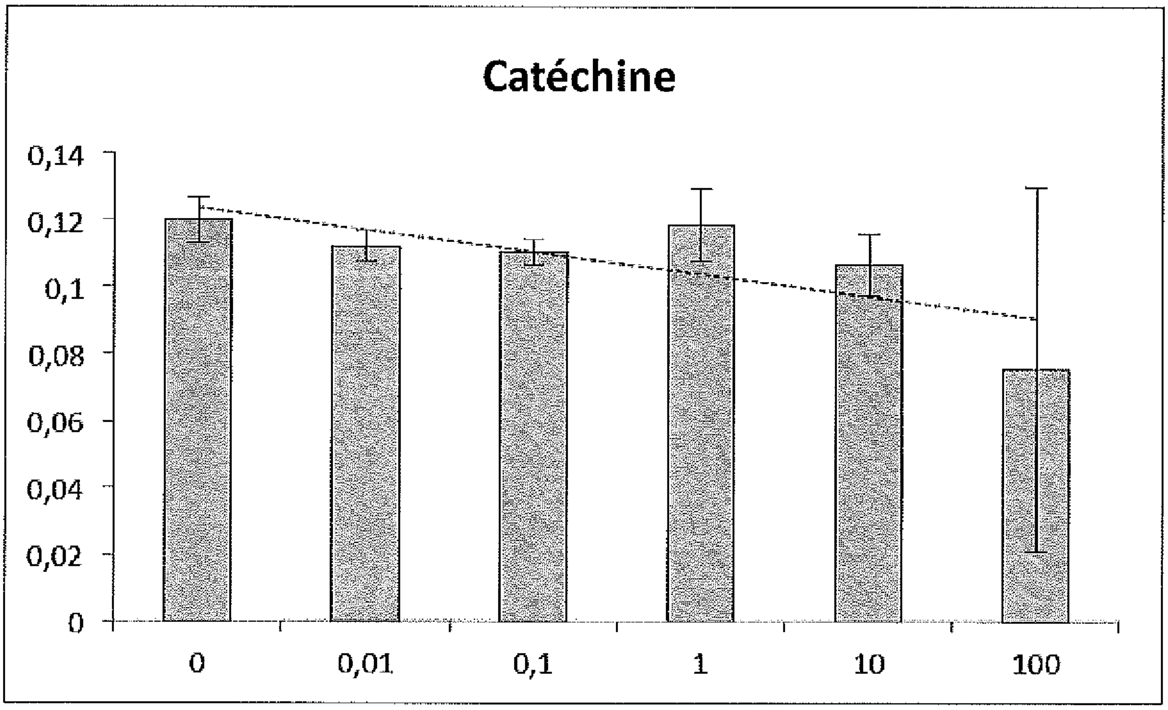


Figure 12

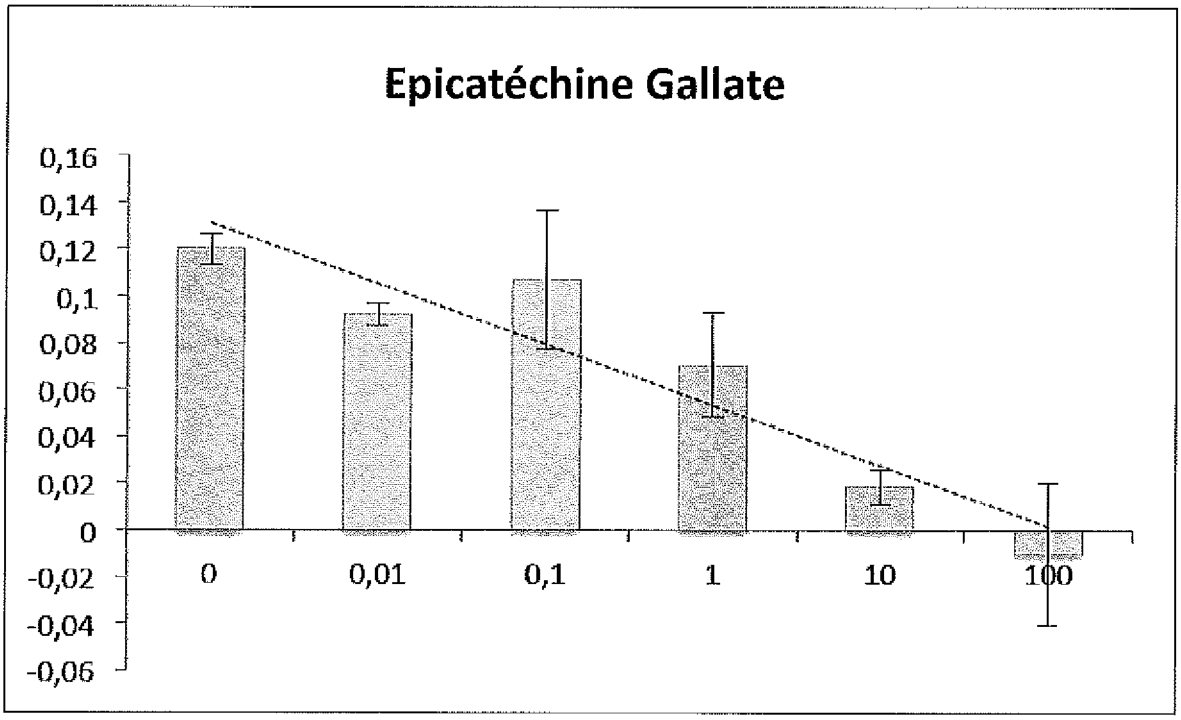


**Figure 13**

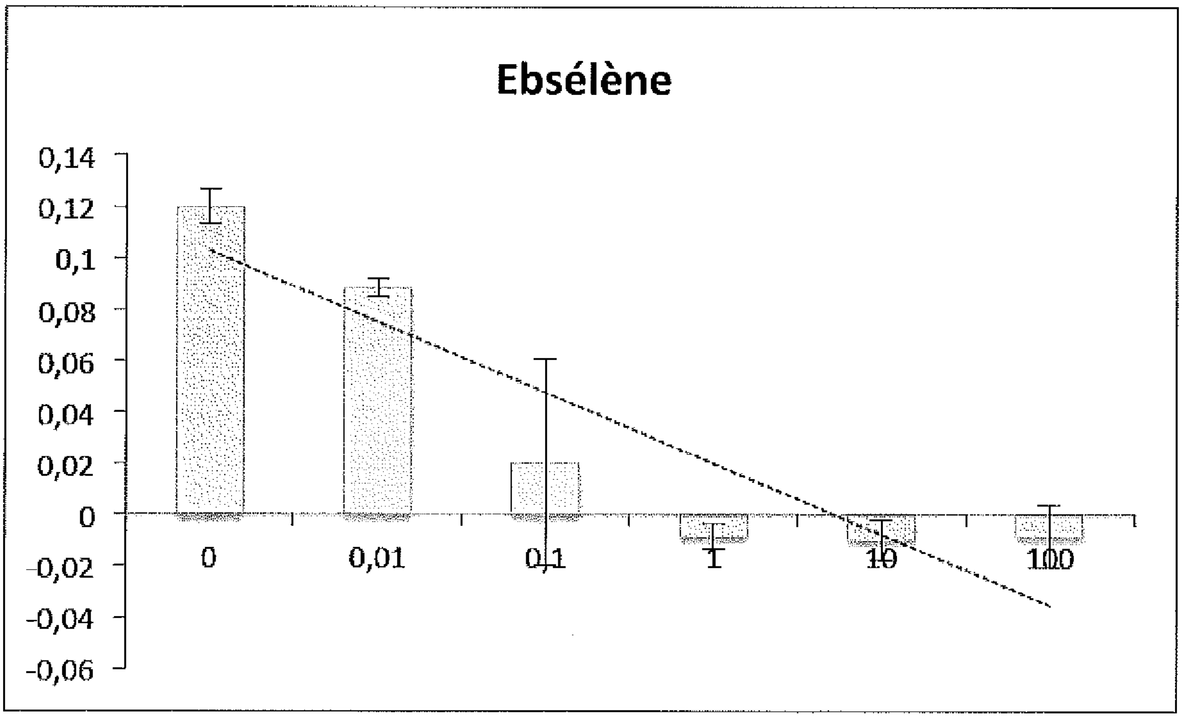


**Figure 14**

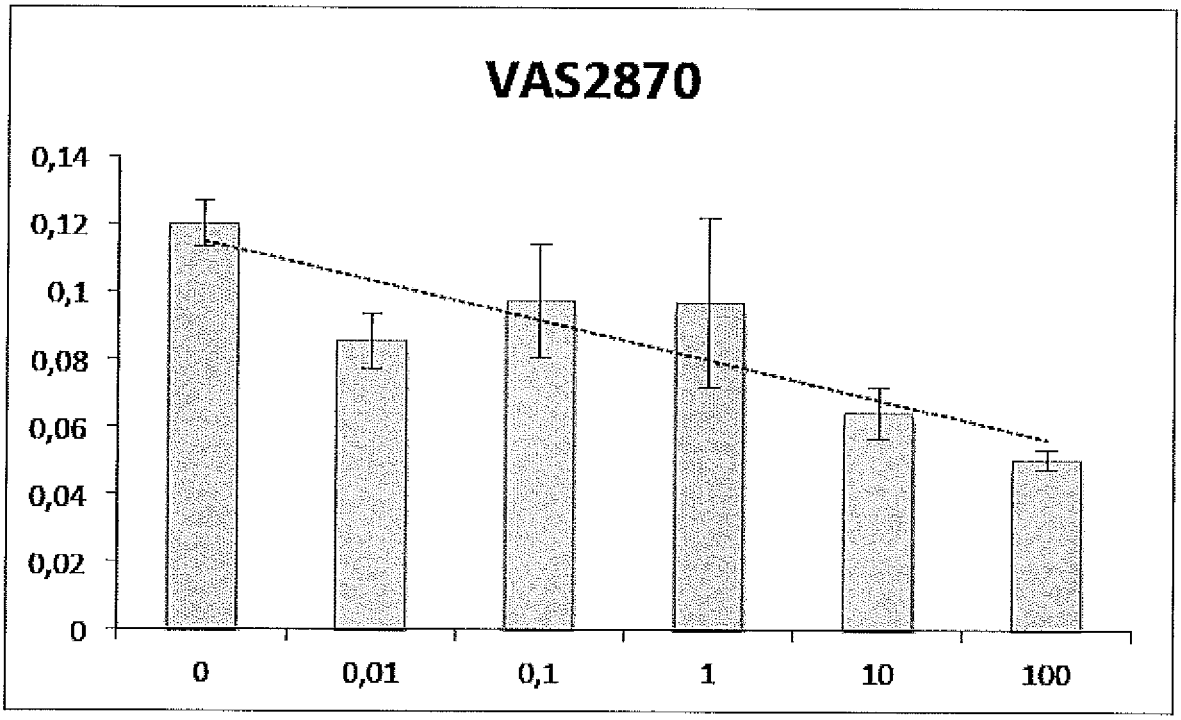


Figure 15

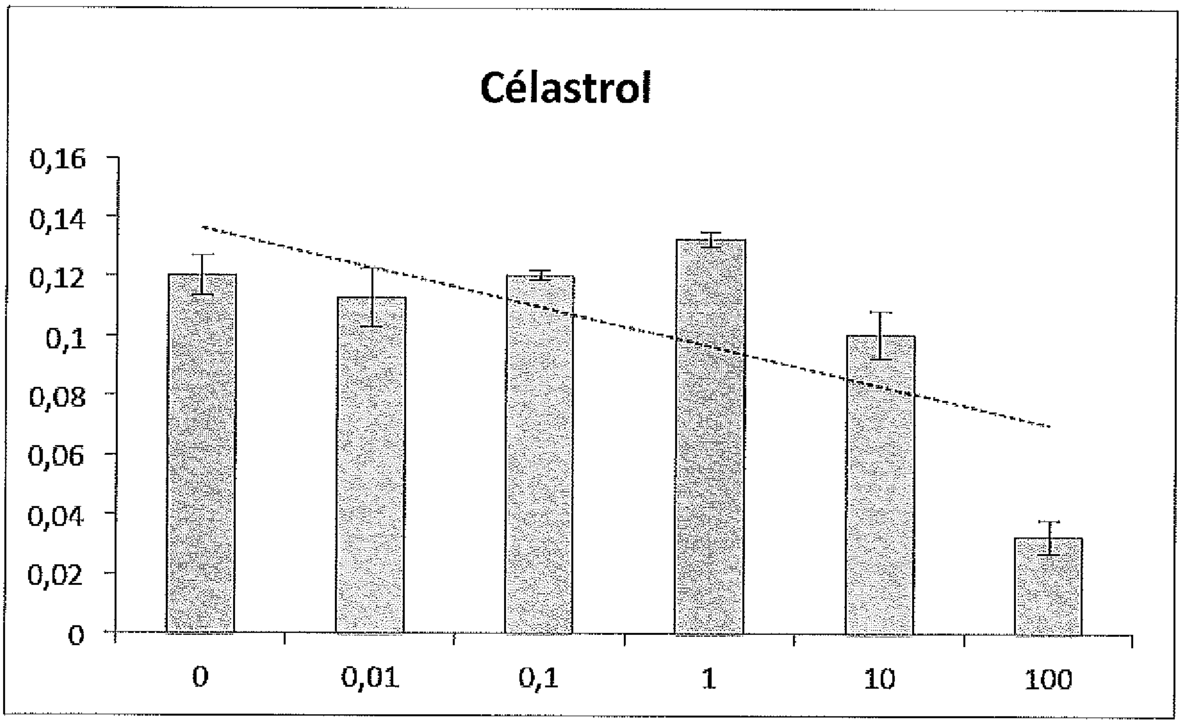


Figure 16

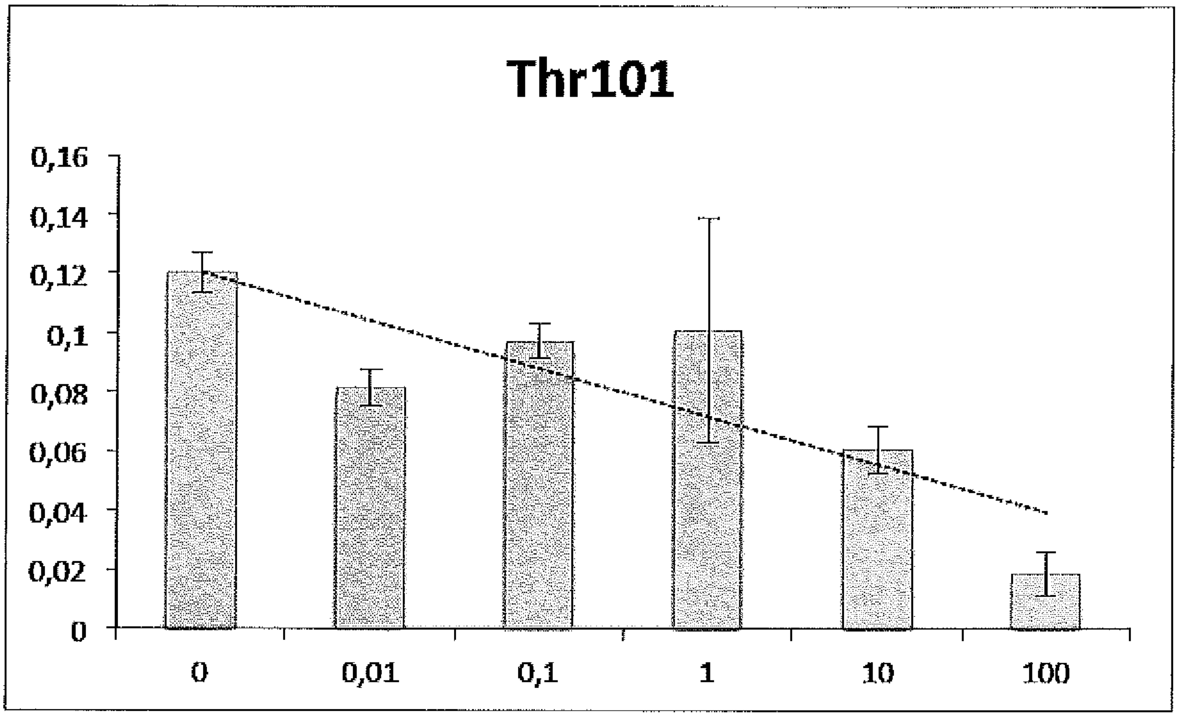


Figure 17

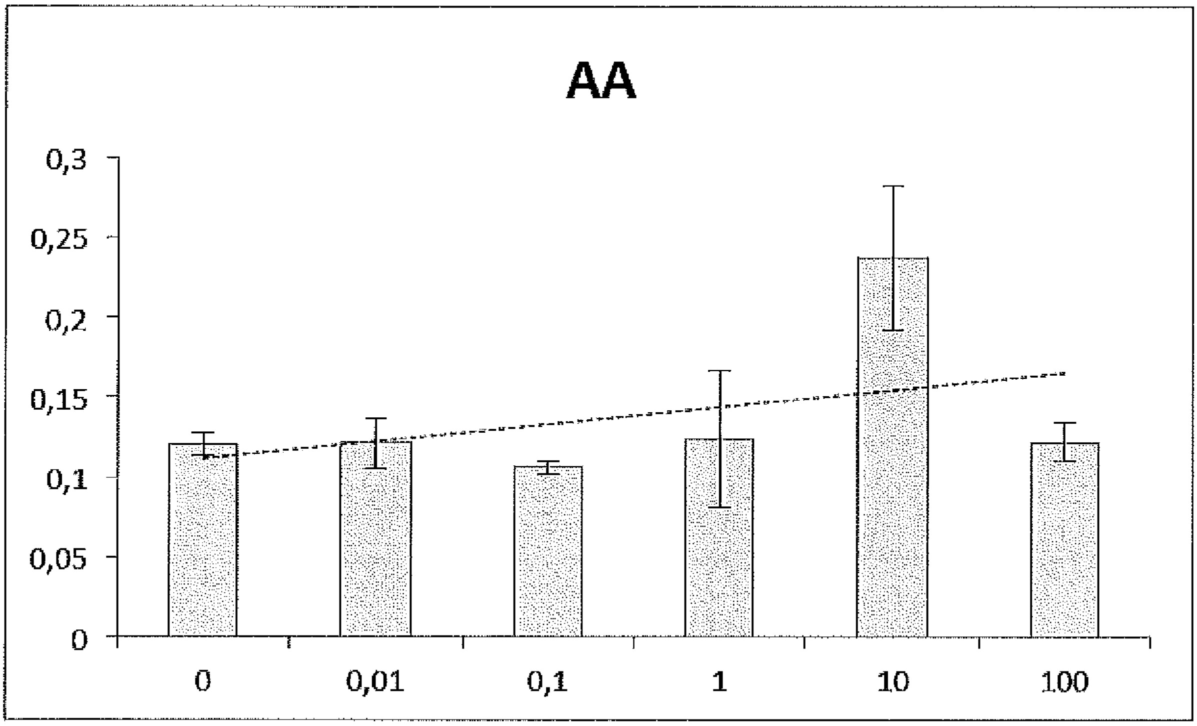


Figure 18

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **LAMBETH JD.** NOX enzymes and the biology of reactive oxygen, Nature reviews. *Immunology,* 2004, vol. 4, 181-189
- **PROSSER BL et al.** X-ROS signaling : rapid mechano-chemo transduction in heart. *Science,* 2011, vol. 333, 1440-1445
- **DE DEKEN X et al.** Cloning of two human thyroid cDNAs encoding new members of the NADPH oxidase family. *The Journal of biological chemistry,* vol. 275, 23227-23233
- **TAYLOR RM et al.** Analysis of human phagocyte flavocytochrome b(558) by mass spectrometry. *J Biol Chem,* 01 Décembre 2006, vol. 281 (48), 37045-56
- **LORD CI et al.** Single-step immunoaffinity purification and functional reconstitution of human phagocyte flavocytochrome b. *J Immunol Methods,* 01 Janvier 2008, vol. 329 (1-2), 201-7
- **DE MENDEZ I ; LETO TL.** Functional reconstitution of the phagocyte NADPH oxidase by transfection of its multiple components in a heterologous system. *Blood,* 15 Février 1995, vol. 85 (4), 1104-10
- **PRICE MO et al.** Creation of a genetic system for analysis of the phagocyte respiratory burst: high-level reconstitution of the NADPH oxidase in a nonhematopoietic system. *Blood,* 15 Avril 2002, vol. 99 (8), 2653-61
- **ZHEN L et al.** Gene targeting of X chromosome-linked chronic granulomatous disease locus in a human myeloid leukemia cell line and rescue by expression of recombinant gp91phox. *Proc Natl Acad Sci USA,* 01 Novembre 1993, vol. 90 (21), 9832-6
- **ROTROSEN D et al.** Production of recombinant cytochrome b558 allows reconstitution of the phagocyte NADPH oxidase solely from recombinant proteins. *J Biol Chem,* 05 Juillet 1993, vol. 268 (19), 14256-60
- **OSTUNI MA et al.** Expression of functional mammal flavocytochrome b(558) in yeast : comparison with improved insect cell system. *Biochim Biophys Acta,* 17 Juin 2010, vol. 98 (6), 1179-88
- **HAN CH et al.** Characterization of the flavoprotein domain of gp91phox which has NADPH diaphorase activity. *J Biochem,* 12 Avril 2001, vol. 9 (4), 513-20
- **NISIMOTO Y et al.** Activation of the flavoprotein domain of gp91phox upon interaction with N-terminal p67phox (1-210) and the Rac complex. *Biochemistry,* 27 Juillet 2004, vol. 43 (29), 9567-75
- **GREEN TR ; WU DE.** Detection of NADPH diaphorase activity associated with human neutrophil NADPH-O2 oxidoreductase activity. *FEBS Lett.,* 01 Janvier 1985, vol. 179 (1), 82-6
- **MIZRAHI et al.** A prenylated p47phox-p67phox-Racl chimera is a Quintessential NADPH oxidase activator: membrane association and functional capacity. *J Biol Chem,* 2010, vol. 285 (33), 25485-99
- **ZHANG et al.** *J Biomol Screen,* 1999, vol. 4 (2), 67-73
- **DAHLGREN, C. ; A. KARLSSON.** Respiratory burst in human neutrophils. *J Immunol Methods,* 1999, vol. 232 (1-2), 3-14
- **MIZRAHI, A. et al.** A prenylated p47phox-p67phox-Rac1 chimera is a Quintessential NADPH oxidase activator: membrane association and functional capacity. *J Biol Chem,* 2010, vol. 285 (33), 25485-99